# EUROPEAN PATENT APPLICATION

(11) **EP 4 207 060 A1**
(43) Date of publication of application: **05.07.2023**
(21) Application number: 22216816.3
(22) Date of filing: 27.12.2022
(51) Int. Cl.: G06T 7/00

(54) **METHODS AND SYSTEMS FOR IMAGE ANALYSIS**

(30) Priority: 27.12.2021 CN 202111612060; 11.05.2022 CN 202210507029
(71) Applicant: Shanghai United Imaging Healthcare Co., Ltd., Shanghai 201807 (CN)
(72) Inventor: SU, Saisai, Shanghai, 201807 (CN); GONG, Zhenhuan, Shanghai, 201807 (CN)
(74) Representative: Wang, Bo

(57) **Abstract**

The present disclosure relates to methods and systems for image analysis. The method may include obtaining a plurality of image sequences relating to a blood vessel. At least one of the plurality of image sequences may be acquired using a black blood imaging sequence. The method may also include determining a plurality of aligned image sequences by aligning, based on a reference image sequence of the plurality of image sequences, the plurality of image sequences, determining a target region based on the plurality of aligned image sequences, and determining one or more target parameters based on the target region.

## Description

### CROSS-REFERENCE TO RELATED APPLICATIONS

This application claims priority to Chinese Patent Application No. 202111612060.5, filed on December 27, 2021, and Chinese Patent Application No. 202210507029.3, filed on May 11, 2022, the entire contents of each of which are hereby incorporated by reference.

### TECHNICAL FIELD

The present disclosure generally relates to medical technology, and more particularly, relates to methods and systems for medical image analysis.

### BACKGROUND

Medical images (e.g., an image relating to a blood vessel) may be acquired by an imaging device (e.g., a magnetic resonance imaging (MRI) device, etc.) using different types of examination. The different types of examination may have different examination objects. For example, the ultrasound examination may be used to obtain blood flow information and determine whether there is a plaque in the blood vessel based on the image of the blood vessel. As another example, the computed tomography angiography (CTA) may be used to obtain parameters of vascular morphology based on the image of the blood vessel. As still another example, the digital subtraction angiography (DSA) may be used to obtain parameters of vascular morphology and provide, by pressure detection using a catheter, vascular pressure information, which is an invasive examination. However, each of the current types of examination cannot perform quantitative analysis and qualitative analysis of the blood vessel simultaneously and the analysis of the blood vessel depends on clinical experience of a user (e.g., an operator, a doctor, a medical technician of the examination), which may affect the accuracy of clinical diagnosis of vascular diseases (e.g., cerebrovascular diseases, atherosclerosis, etc.) and/or diagnostic efficiency.

Thus, it is desirable to provide a method and system for medical image analysis, which can perform comprehensive analysis on vascular images, thereby improving the accuracy and/or efficiency of the medical image analysis.

### SUMMARY

According to an aspect of the present disclosure, a method for image analysis is provided. The method may include obtaining a plurality of image sequences relating to a blood vessel. At least one of the plurality of image sequences may be acquired using a black blood imaging sequence. The method may also include determining a plurality of aligned image sequences by aligning, based on a reference image sequence of the plurality of image sequences, the plurality of image sequences, determining a target region based on the plurality of aligned image sequences, and determining one or more target parameters based on the target region.

In some embodiments, the target region may include a region of a target tissue and a reference region of interest (ROI). The one or more target parameters may include an enhancement level of the target tissue. The determining the one or more target parameters based on the target region may include determining, based on the region of the target tissue, a first enhancement degree of the region of the target tissue, determining, based on the reference ROI, a second enhancement degree of the reference ROI, and determining the enhancement level of the target tissue based on the first enhancement degree and the second enhancement degree.

In some embodiments, the determining, based on the region of the target tissue, the first enhancement degree of the region of the target tissue may include determining a first intensity of the region of the target tissue before enhancement, determining a second intensity of the region of the target tissue after enhancement, and determining, based on the first intensity and the second intensity, the first enhancement degree of the region of the target tissue.

In some embodiments, the determining, based on the refence ROI, the second enhancement degree of the reference ROI may include determining a third intensity of the reference ROI before enhancement, determining a fourth intensity of the reference ROI after enhancement, and determining, based on the third intensity and the fourth intensity, the second enhancement degree of the reference ROI.

In some embodiments, the target tissue may include a vascular wall and a plaque, and the target tissue may be determined by determining the vascular wall by identifying the vascular wall from the plurality of aligned image sequences, and determining the plaque by identifying the plaque from the vascular wall.

In some embodiments, the reference ROI may be determined by inputting the plurality of aligned image sequences into a segmentation model.

In some embodiments, the determining the enhancement level of the target tissue based on the first enhancement degree and the second enhancement degree may include determining the enhancement level by dividing the first enhancement degree by the second enhancement degree.

In some embodiments, the target region may include a region of a target blood vessel, and the one or more target parameters may include at least one of a flow quantity of the target blood vessel, a flow rate of the blood vessel, a wall thickness of the target blood vessel, or a wall shear force of the target blood vessel.

In some embodiments, the plurality of aligned image sequences may include a first aligned image sequence and a second aligned image sequence, and the determining the one or more target parameters based on the target region may include determining at least one region including the target blood vessel by processing the first aligned image sequence and the second aligned image sequence, determining blood flow information and wall information of the target blood vessel based on the at least one region including the target blood vessel, and determining the one or more target parameters based on the blood flow information and the wall information.

In some embodiments, the determining the one or more target parameters based on the blood flow information and the wall information may include determining a reconstructed image of the target blood vessel based on the blood flow information and the wall information, and determining the one or more target parameters based on the reconstructed image.

In some embodiments, the method may further include determining a first plane of the target blood vessel based on the blood flow information and the wall information, and determining one or more parameters of the first plane based on the first plane and the one or more target parameters.

In some embodiments, the determining the one or more target parameters based on the blood flow information and the wall information may include extracting a blood flow mask, a lumen mask, and a wall mask from the blood flow information and the wall information, and determining the one or more target parameters based on the blood flow mask, the lumen mask, and the wall mask.

According to another aspect of the present disclosure, a system for image analysis is provided. The system may include an obtaining module configured to obtain a plurality of image sequences relating to a blood vessel. At least one of the plurality of image sequences may be acquired using a black blood imaging sequence. The system may also include an aligning module configured to determine a plurality of aligned image sequences by aligning, based on a reference image sequence of the plurality of image sequences, the plurality of image sequences. The system may also include a determining module, configured to determine a target region based on the plurality of aligned image sequences. The system may further include the determining module configured to determine one or more target parameters based on the target region.

According to yet another aspect of the present disclosure, a non-transitory computer readable medium including a set of instructions for generating a 3D image is provided. When executed by at least one processor, the set of instructions may direct the at least one processor to effectuate a method, the method may include obtaining a plurality of image sequences relating to a blood vessel. At least one of the plurality of image sequences may be acquired using a black blood imaging sequence. The method may also include determining a plurality of aligned image sequences by aligning, based on a reference image sequence of the plurality of image sequences, the plurality of image sequences, determining a target region based on the plurality of aligned image sequences, and determining one or more target parameters based on the target region.

Additional features will be set forth in part in the description which follows, and in part will become apparent to those skilled in the art upon examination of the following and the accompanying drawings or may be learned by production or operation of the examples. The features of the present disclosure may be realized and attained by practice or use of various aspects of the methodologies, instrumentalities, and combinations set forth in the detailed examples discussed below.

### BRIEF DESCRIPTION OF THE DRAWINGS

The present disclosure is further described in terms of exemplary embodiments. These exemplary embodiments are described in detail with reference to the drawings. The drawings are not to scale. These embodiments are non-limiting exemplary embodiments, in which like reference numerals represent similar structures throughout the several views of the drawings, and wherein:
FIG. 1 is a schematic diagram illustrating an exemplary image analysis system according to some embodiments of the present disclosure;
FIG. 2 is a schematic diagram illustrating hardware and/or software components of an exemplary computing device according to some embodiments of the present disclosure;
FIG. 3 is a schematic diagram illustrating hardware and/or software components of an exemplary mobile device according to some embodiments of the present disclosure;
FIG. 4 is a block diagram illustrating an exemplary processing device according to some embodiments of the present disclosure;
FIG. 5 is a flowchart illustrating an exemplary process for image analysis according to some embodiments of the present disclosure;
FIG. 6 is a flowchart illustrating an exemplary process for determining an enhancement level of a target tissue according to some embodiments of the present disclosure;
FIG. 7 is a flowchart illustrating an exemplary process for determining a target tissue according to some embodiments of the present disclosure;
FIG. 8 is a flowchart illustrating an exemplary process for determining a reference ROI according to some embodiments of the present disclosure;
FIG. 9 is a flowchart illustrating an exemplary training process of a segmentation model according to some embodiments of the present disclosure;
FIG. 10 is a flowchart illustrating an exemplary process for a determination of an enhancement level according to some embodiments of the present disclosure;
FIG. 11 is a flowchart illustrating an exemplary process for a determination of a plurality of aligned image sequences according to some embodiments of the present disclosure;
FIG. 12 is a schematic diagram illustrating an exemplary layout of a plurality of aligned image sequences according to some embodiments of the present disclosure;
FIG. 13 is a schematic diagram illustrating an exemplary report of a target tissue according to some embodiments of the present disclosure;
FIG. 14 is a flowchart illustrating an exemplary process for a determination of one or more target parameters according to some embodiments of the present disclosure;
FIG. 15 is schematic diagram illustrating an exemplary process for a determination of one or more target parameters according to some embodiments of the present disclosure;
FIG. 16 is schematic diagram illustrating an exemplary process for a determination of a parameter of a first plane according to some embodiments of the present disclosure;
FIG. 17 is schematic diagram illustrating an exemplary reconstructed image according to some embodiments of the present disclosure;
FIG. 18 is a flowchart illustrating an exemplary process for image analysis according to some embodiments of the present disclosure; and
FIG. 19 is a flowchart illustrating an exemplary process for image analysis according to some embodiments of the present disclosure.

### DETAILED DESCRIPTION

The following description is presented to enable any person skilled in the art to make and use the present disclosure and is provided in the context of a particular application and its requirements. Various modifications to the disclosed embodiments will be readily apparent to those skilled in the art, and the general principles defined herein may be applied to other embodiments and applications without departing from the spirit and scope of the present disclosure. Thus, the present disclosure is not limited to the embodiments shown but is to be accorded the widest scope consistent with the claims.

The terminology used herein is for the purpose of describing particular example embodiments only and is not intended to be limiting. As used herein, the singular forms "a," "an," and "the" may be intended to include the plural forms as well, unless the context clearly indicates otherwise. It will be further understood that the terms "comprise," "comprises," and/or "comprising," "include," "includes," and/or "including" when used in this disclosure, specify the presence of stated features, integers, steps, operations, elements, and/or components, but do not preclude the presence or addition of one or more other features, integers, steps, operations, elements, components, and/or groups thereof.

Generally, the word "module," "unit," or "block," as used herein, refers to logic embodied in hardware or firmware, or to a collection of software instructions. A module, a unit, or a block described herein may be implemented as software and/or hardware and may be stored in any type of non-transitory computer-readable medium or other storage devices. In some embodiments, a software module/unit/block may be compiled and linked into an executable program. It will be appreciated that software modules can be callable from other modules/units/blocks or from themselves, and/or may be invoked in response to detected events or interrupts. Software modules/units/blocks configured for execution on computing devices may be provided on a computer-readable medium, such as a compact disc, a digital video disc, a flash drive, a magnetic disc, or any other tangible medium, or as a digital download (and can be originally stored in a compressed or installable format that needs installation, decompression, or decryption prior to execution). Such software code may be stored, partially or fully, on a storage device of the executing computing device, for execution by the computing device. Software instructions may be embedded in firmware, such as an erasable programmable read-only memory (EPROM). It will be further appreciated that hardware modules/units/blocks may be included in connected logic components, such as gates and flip-flops, and/or can be included of programmable units, such as programmable gate arrays or processors. The modules/units/blocks or computing device functionality described herein may be implemented as software modules/units/blocks but may be represented in hardware or firmware. In general, the modules/units/blocks described herein refer to logical modules/units/blocks that may be combined with other modules/units/blocks or divided into sub-modules/sub-units/sub-blocks despite their physical organization or storage. The description may be applicable to a system, an engine, or a portion thereof.

It will be understood that the term "system," "engine," "unit," "module," and/or "block" used herein are one method to distinguish different components, elements, parts, sections or assembly of different levels in ascending order. However, the terms may be displaced by another expression if they achieve the same purpose.

It will be understood that when a unit, engine, module, or block is referred to as being "on," "connected to," or "coupled to," another unit, engine, module, or block, it may be directly on, connected or coupled to, or communicate with the other unit, engine, module, or block, or an intervening unit, engine, module, or block may be present, unless the context clearly indicates otherwise. As used herein, the term "and/or" includes any and all combinations of one or more of the associated listed items.

The term "pixel" and "voxel" in the present disclosure may be used interchangeably to refer to an element in an image. The term "image" in the present disclosure may be used to refer to images of various forms, including a 2-dimensional (2D) image, a 3-dimensional (3D) image, a 4-dimensional (4D) image, etc.

In the present disclosure, a representation of an object (e.g., a patient, a subject, or a portion thereof) in an image may be referred to as an "object" for brevity. For instance, a representation of an organ or tissue (e.g., a heart, a liver, a lung) in an image may be referred to as an organ or tissue for brevity. Further, an image including a representation of an object may be referred to as an image of an object or an image including an object for brevity. Still further, an operation performed on a representation of an object in an image may be referred to as an operation performed on an object for brevity. For instance, a segmentation of a portion of an image including a representation of an organ or tissue from the image may be referred to as a segmentation of an organ or tissue for brevity.

These and other features, and characteristics of the present disclosure, as well as the methods of operation and functions of the related elements of structure and the combination of parts and economies of manufacture, may become more apparent upon consideration of the following description with reference to the accompanying drawings, all of which form a part of this disclosure. It is to be expressly understood, however, that the drawings are for the purpose of illustration and description only and are not intended to limit the scope of the present disclosure. It is understood that the drawings are not to scale.

The flowcharts used in the present disclosure illustrate operations that systems implement according to some embodiments in the present disclosure. It is to be expressly understood, the operations of the flowchart may be implemented not in order. Conversely, the operations may be implemented in an inverted order, or simultaneously. Moreover, one or more other operations may be added to the flowcharts. One or more operations may be removed from the flowcharts.

Provided herein are systems and components for medical imaging and/or medical treatment. In some embodiments, the medical system may include an imaging system. The imaging system may include a single modality imaging system and/or a multi-modality imaging system. The single modality imaging system may include, for example, a magnetic resonance imaging (MRI) system, a computed tomography (CT) system, a digital subtraction angiography (DSA) system, an intravascular ultrasound (IVUS) device, etc. that can perform vascular imaging. Exemplary MRI systems may include a superconducting magnetic resonance imaging system, a non-superconducting magnetic resonance imaging system, etc. The multi-modality imaging system may include, for example, a magnetic resonance-computed tomography imaging (MRI-CT) system, a positron emission tomography-magnetic resonance imaging (PET-MRI) system, a single photon emission computed tomography-magnetic resonance imaging (SPECT-MRI) system, a digital subtraction angiography-magnetic resonance imaging (DSA-MRI) system, , a digital subtraction angiography-computed tomography (DSA-CT) system, a single photon emission computed tomography-computed tomography (SPECT-CT) system, a digital subtraction angiography-positron emission tomography (DSA-PET) system etc. In some embodiments, the medical system may include a treatment system. The treatment system may include a treatment plan system (TPS), image-guide radiotherapy (IGRT), etc. The image-guide radiotherapy (IGRT) may include a treatment device and an imaging device. The treatment device may include a linear accelerator, a cyclotron, a synchrotron, etc., configured to perform a radio therapy on a subject. The treatment device may include an accelerator of species of particles including, for example, photons, electrons, protons, or heavy ions.

The term "imaging modality" or "modality" as used herein broadly refers to an imaging method or technology that gathers, generates, processes, and/or analyzes imaging information of an object. The object may include a biological object and/or a non-biological object. The biological object may be a human being, an animal, a plant, or a portion thereof (e.g., a cell, a tissue, an organ, etc.). In some embodiments, the object may be a man-made composition of organic and/or inorganic matters that are with or without life. The term "object" or "subject" are used interchangeably.

An aspect of the present disclosure relates to systems and methods for image analysis. The system may obtain a plurality of image sequences relating to a blood vessel. At least one of the plurality of image sequences may be acquired using a black blood imaging sequence. The system may also determine a plurality of aligned image sequences by aligning the plurality of image sequences based on a reference image sequence of the plurality of image sequences. The system may also determine a target region based on the plurality of aligned image sequences. The system may further determine one or more target parameters based on the target region.

According to some embodiments of the present disclosure, the plurality of aligned image sequences may allow viewing the plurality of image sequences of the blood vessel at the same time, and comprehensive information about the blood vessel may be determined by performing a quantitative and qualitative analysis on the plurality of aligned image sequences, thereby improving an accuracy analysis of a target tissue (e.g., a plaque, an aneurysm, a stenosis plane, etc.) in the blood vessel. In addition, one or more target parameters may be determined based on the target region determined based on the plurality of aligned image sequences, thereby avoiding manual intervention in analysis process and improving an accuracy of determining the target parameters (e.g., an enhancement level of a target tissue included in the target region).

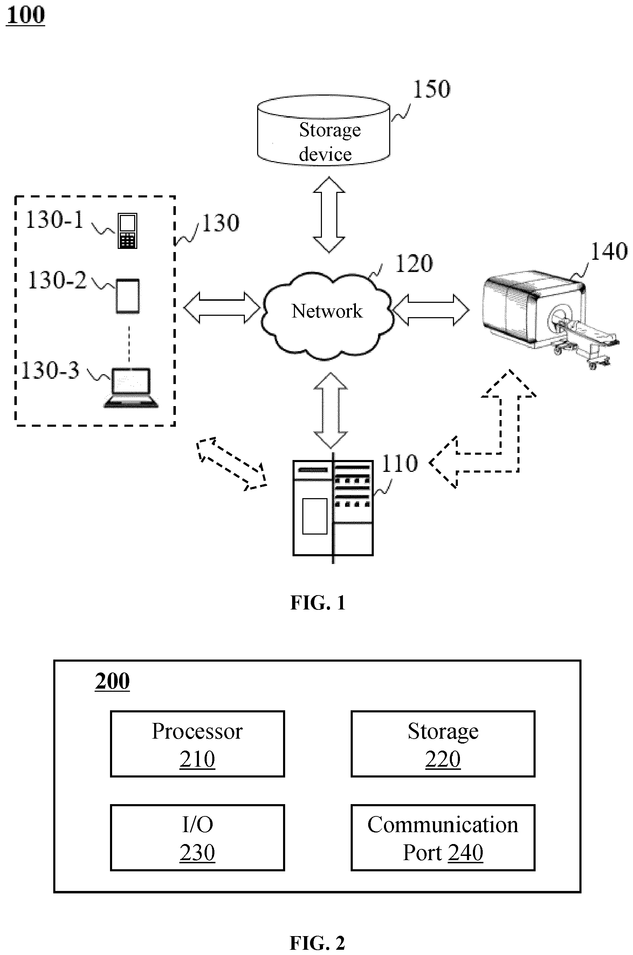

FIG. 1 is a schematic diagram illustrating an exemplary image analysis system according to some embodiments of the present disclosure. As illustrated, the image analysis system 100 may include a processing device 110, a network 120, a terminal 130, an imaging device 140, and a storage device 150. The components of the image analysis system 100 may be connected in one or more of various ways. Merely by way of example, as illustrated in FIG. 1, the imaging device 140 may be connected to the processing device 110 through the network 120. As another example, the imaging device 140 may be connected to the processing device 110 directly (as indicated by the bi-directional arrow in dotted lines linking the imaging device 140 and the processing device 110). As a further example, the storage device 150 may be connected to the processing device 110 directly or through the network 120. As still a further example, a terminal device (e.g., 130-1, 130-2, 130-3, etc.) may be connected to the processing device 110 directly (as indicated by the bi-directional arrow in dotted lines linking the terminal 130 and the processing device 110) or through the network 120.

In some embodiments, the processing device 110 may process data and/or information obtained from one or more components (e.g., the terminal 130, the imaging device 140, and/or the storage device 150) of the image analysis system 100. The processing device 110 may perform one or more processes disclosed in the present disclosure for determining one or more target parameters. For example, the processing device 110 may obtain a plurality of image sequences relating to a blood vessel from, e.g., the imaging device 140 or the storage device 150. As another example, the processing device 110 may determine one or more target parameters by processing the plurality of image sequences. For instance, the processing device 110 may determine a plurality of aligned image sequences by aligning, based on a reference image sequence of the plurality of image sequences, the plurality of image sequences. The processing device 110 may determine a target region based on the plurality of aligned image sequences. The processing device 110 may determine the one or more target parameters based on the target region. As another example, the processing device 110 may transmit the one or more target parameters to one or more components (e.g., the terminal 130, the imaging device 140, or the storage device 150) of image analysis system 100 for display and/or storage. In some embodiments, the processing device 110 may be a single server or a server group. The server group may be centralized or distributed. In some embodiments, the processing device 110 may be local or remote. In some embodiments, the processing device 110 may be implemented on a cloud platform.

In some embodiments, the processing device 110 may include one or more sub-processing devices (e.g., a single-core processing device, a multi-core processing deivce, etc.). Merely by way of example, the processing device 110 may include a central processing unit (CPU), an application-specific integrated circuits (ASICs), an application-specific instruction-set processor (ASIP), a graphics processing unit (GPU), a physics processing unit (PPU), a digital signal processor (DSP), a field-programmable gate array (FPGA), a programmable logic device (PLD), a controller, a microcontroller, a reduced instruction set computer (RISC), a microprocessor, or the like, or any combinations thereof.

The network 120 may include any suitable network that can facilitate the exchange of information and/or data for the image analysis system 100. In some embodiments, one or more components (e.g., the processing device 110, the terminal 130, the imaging device 140, or the storage device 150) of the image analysis system 100 may communicate information and/or data with one or more other components of the image analysis system 100 via the network 120. For example, the processing device 110 may obtain the plurality of image sequences from the imaging device 140 via the network 120. In some embodiments, the network 120 may be any type of wired or wireless network, or a combination thereof.

The terminal 130 may include a mobile device 130-1, a tablet computer 130-2, a laptop computer 130-3, or the like, or any combination thereof. In some embodiments, the mobile device 130-1 may include a smart home device, a wearable device, a smart mobile device, a virtual reality device, an augmented reality device, or the like, or any combination thereof. In some embodiments, the terminal 130 may remotely operate the imaging device 140 and/or the processing device 110. In some embodiments, the terminal 130 may operate the imaging device 140 and/or the processing device 110 via a wireless connection. In some embodiments, the terminal 130 may receive information and/or instructions inputted by a user, and send the received information and/or instructions to the imaging device 140 or to the processing device 110 via the network 120. In some embodiments, the terminal 130 may receive data and/or information from the processing device 110. In some embodiments, the terminal 130 may be part of the processing device 110. In some embodiments, the terminal 130 may be omitted.

The imaging device 140 may scan a subject located within its detection region and generate data relating to the subject. In the present disclosure, "subject" and "object" are used interchangeably. Merely by way of example, the subject may include a patient, a man-made subject, etc. As another example, the subject may include a specific portion, organ, and/or tissue of a patient. For example, the subject may include head, brain, neck, body, shoulder, arm, thorax, cardiac, stomach, blood vessel, soft tissue, knee, feet, or the like, or any combination thereof. In some embodiments, the imaging device 140 may include, for example, an MRI device, a CT device, an X-ray imaging device (e.g., a DSA device), an IVUS device, a molecular imaging device (e.g., a PET device or a SPECT device), or the like, or any combination thereof. In some embodiments, the imaging device 140 may obtain the image data relating to the blood vessel using a magnetic resonance angiography (MRA) technology, a computed tomography angiography (CTA) technology, a 4D flow MRI technology (e.g., a 3D phase contrast MRI technology with time resolution). For illustration purposes, the following description is described with reference to an MRI device unless specifically stated.

The storage device 150 may store data and/or instructions. In some embodiments, the storage device 150 may store data obtained from the imaging device 140, the terminal 130, and/or the processing device 110. For example, the storage device 150 may store the plurality of image sequences which are acquired by the imaging device 140 in a scan and/or the plurality of aligned image sequences generated based on the plurality of image sequences. In some embodiments, the storage device 150 may store data and/or instructions that the processing device 110 may execute or use to perform exemplary methods described in the present disclosure. For example, the storage device 150 may store instructions that the processing device 110 may execute to determine the one or more target parameters. In some embodiments, the storage device 150 may include a mass storage device, a removable storage device, a volatile read-and-write memory, a read-only memory (ROM), or the like, or any combination thereof. For example, the mass storage device may include a disk, an optical disk, a solid state disk, etc. In some embodiments, the storage device 150 may be implemented on a cloud platform.

In some embodiments, the storage device 150 may be connected to the network 120 to communicate with one or more components (e.g., the processing device 110, the terminal 130, the imaging device 140, etc.) of the image analysis system 100. One or more components of the image analysis system 100 may access the data or instructions stored in the storage device 150 via the network 120. In some embodiments, the storage device 150 may be part of the processing device 110. It should be noted that the above description of the image analysis system 100 is merely provided for the purposes of illustration, and not intended to limit the scope of the present disclosure. For persons having ordinary skills in the art, multiple variations and modifications may be made under the teachings of the present disclosure. In some embodiments, the image analysis system 100 may include one or more additional components and/or one or more components of the image analysis system 100 described above may be omitted. For example, the image analysis system 100 may further include one or more power supplies (not shown in FIG. 1) operably connected to one or more components of the image analysis system 100 (e.g., the processing device 110, the terminal 130, the imaging device 140, the storage device 150, etc.). Additionally or alternatively, two or more components of the image analysis system 100 may be integrated into a single component. A component of the image analysis system 100 may be implemented on two or more sub-components. In some embodiments, the image analysis system 100 may further include one or more power.

FIG. 2 is a schematic diagram illustrating hardware and/or software components of an exemplary computing device 200 according to some embodiments of the present disclosure. The computing device 200 may be used to implement any component of the image analysis system 100 as described herein. For example, the processing device 110 and/or the terminal 130 may be implemented on the computing device 200, respectively, via its hardware, software program, firmware, or a combination thereof. Although only one such computing device is shown, for convenience, the computer functions relating to the image analysis system 100 as described herein may be implemented in a distributed fashion on a number of similar platforms, to distribute the processing load. As illustrated in FIG. 2, the computing device 200 may include a processor 210, a storage 220, an input/output (I/O) 230, and a communication port 240.

The processor 210 may execute computer instructions (program codes) and perform functions of the processing device 110 in accordance with techniques described herein. The computer instructions may include, for example, routines, programs, objects, components, signals, data structures, procedures, modules, and functions, which perform particular functions described herein. In some embodiments, the processor 210 may perform instructions obtained from the terminal(s) 130. In some embodiments, the processor 210 may include one or more hardware processors, such as a microcontroller, a microprocessor, a reduced instruction set computer (RISC), an application-specific integrated circuits (ASICs), an application-specific instruction-set processor (ASIP), a central processing unit (CPU), a graphics processing unit (GPU), a physics processing unit (PPU), a microcontroller unit, a digital signal processor (DSP), a field-programmable gate array (FPGA), an advanced RISC machine (ARM), a programmable logic device (PLD), any circuit or processor capable of executing one or more functions, or the like, or any combinations thereof.

Merely for illustration, only one processor is described in the computing device 200. However, it should be noted that the computing device 200 in the present disclosure may also include multiple processors. Thus operations and/or method steps that are performed by one processor as described in the present disclosure may also be jointly or separately performed by the multiple processors. For example, if in the present disclosure the processor of the computing device 200 executes both operation A and operation B, it should be understood that operation A and operation B may also be performed by two or more different processors jointly or separately in the computing device 200 (e.g., a first processor executes operation A and a second processor executes operation B, or the first and second processors jointly execute operations A and B).

The storage 220 may store data/information obtained from the imaging device 140, the terminal(s) 130, the storage device 150, or any other component of the image analysis system 100. In some embodiments, the storage 220 may include a mass storage device, a removable storage device, a volatile read-and-write memory, a read-only memory (ROM), or the like, or any combination thereof. In some embodiments, the storage 220 may store one or more programs and/or instructions to perform exemplary methods described in the present disclosure. For example, the storage 220 may store a program for the processing device 110 for performing image processing, such as, image determination, vascular centerline determination, boundary determination, or labeled centerline determination.

The I/O 230 may input or output signals, data, and/or information. In some embodiments, the I/O 230 may enable user interaction with the processing device 110. In some embodiments, the I/O 230 may include an input device and an output device. Exemplary input devices may include a keyboard, a mouse, a touch screen, a microphone, or the like, or a combination thereof. Exemplary output devices may include a display device, a loudspeaker, a printer, a projector, or the like, or a combination thereof. Exemplary display devices may include a liquid crystal display (LCD), a light-emitting diode (LED)-based display, a flat panel display, a curved screen, a television device, a cathode ray tube (CRT), or the like, or a combination thereof.

The communication port 240 may be connected with a network (e.g., the network 120) to facilitate data communications. The communication port 240 may establish connections between the processing device 110 and the imaging device 140, the terminal(s) 130, or the storage device 150. The connection may be a wired connection, a wireless connection, or a combination of both that enables data transmission and reception. The wired connection may include an electrical cable, an optical cable, a telephone wire, or the like, or any combination thereof. The wireless connection may include a Bluetooth^{™} network, a Wi-Fi network, a WiMax network, a WLAN, a ZigBee^{™} network, a mobile network (e.g., 3G, 4G, 5G, etc.), or the like, or any combination thereof. In some embodiments, the communication port 240 may be a standardized communication port, such as RS232, RS485, etc. In some embodiments, the communication port 240 may be a specially designed communication port. For example, the communication port 240 may be designed in accordance with the digital imaging and communications in medicine (DICOM) protocol.

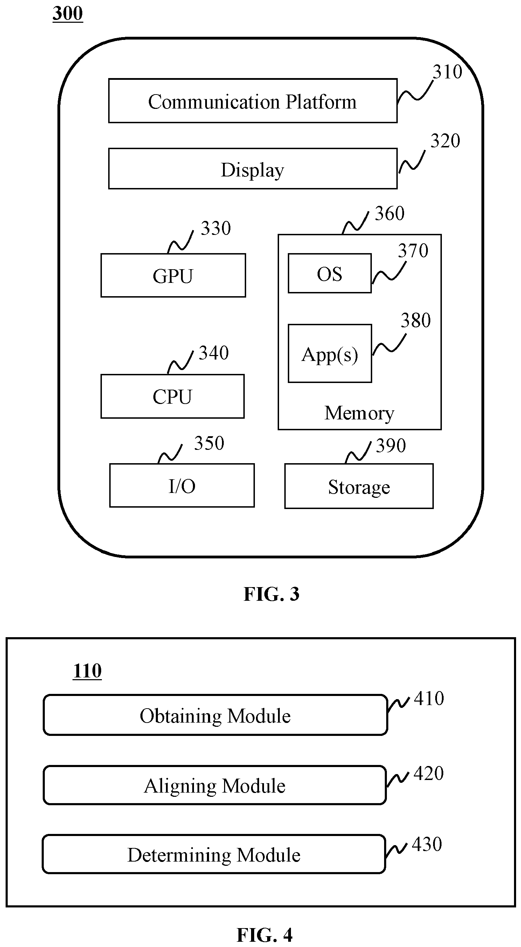

FIG. 3 is a schematic diagram illustrating hardware and/or software components of an exemplary mobile device 300 according to some embodiments of the present disclosure. In some embodiments, one or more components (e.g., a terminal 130 and/or the processing device 110) of the image analysis system 100 may be implemented on the mobile device 300.

As illustrated in FIG. 3, the mobile device 300 may include a communication platform 310, a display 320, a graphics processing unit (GPU) 330, a central processing unit (CPU) 340, an I/O 350, a memory 360, and a storage 390. In some embodiments, any other suitable component, including but not limited to a system bus or a controller (not shown), may also be included in the mobile device 300. In some embodiments, a mobile operating system 370 (e.g., iOS, Android, Windows Phone, etc.) and one or more applications 380 may be loaded into the memory 360 from the storage 390 in order to be executed by the CPU 340. The applications 380 may include a browser or any other suitable mobile apps for receiving and rendering information relating to image processing or other information from the processing device 110. User interactions with the information stream may be achieved via the I/O 350 and provided to the processing device 110 and/or other components of the image analysis system 100 via the network 120.

To implement various modules, units, and their functionalities described in the present disclosure, computer hardware platforms may be used as the hardware platform(s) for one or more of the elements described herein. The hardware elements, operating systems, and programming languages of such computers are conventional in nature, and it is presumed that those skilled in the art are adequately familiar therewith to adapt those technologies to generate an image as described herein. A computer with user interface elements may be used to implement a personal computer (PC) or another type of work station or terminal device, although a computer may also act as a server if appropriately programmed. It is believed that those skilled in the art are familiar with the structure, programming, and general operation of such computer equipment and as a result, the drawings should be self-explanatory.

FIG. 4 is a schematic block diagram illustrating an exemplary processing device according to some embodiments of the present disclosure. The processing device 110 may include an obtaining module 410, an aligning module 420, and a determining module 430.

The obtaining module 410 may be configured to obtain data/information from one or more components of the image analysis system 100. For example, the obtaining module 410 may obtain a plurality of image sequences relating to a blood vessel from the imaging device 140 or a storage device (e.g., the storage device 150 or an external storage device of the imaging analysis system 100). More descriptions of obtaining the plurality of image sequences relating to the blood vessel may be found elsewhere in the present disclosure (e.g., operation 510 or the descriptions thereof).

The aligning module 420 may be configured to perform an image alignment operation on the plurality of image sequences. For example, the aligning module 420 may determine a plurality of aligned image sequences by aligning the plurality of image sequences based on a reference image sequence of the plurality of image sequences. More descriptions of aligning the plurality of image sequences may be found elsewhere in the present disclosure (e.g., operation 520 or the descriptions thereof).

The determining module 430 may be configured to determine a target region based on the plurality of aligned image sequences. In some embodiments, the determining module 430 may be configured to determine one or more target parameters based on the target region. More descriptions of determining of the target region and one or more target parameters may be found elsewhere in the present disclosure (e.g., operations 530 and 540 or relevant descriptions thereof).

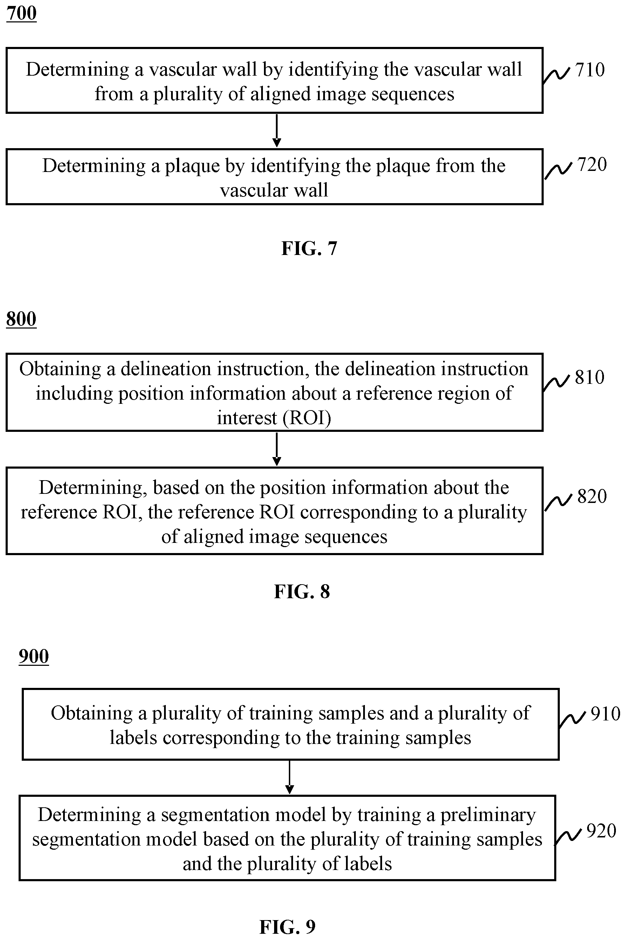

In some embodiments, the processing device 140 may include a training module (not shown in FIG. 4). The training module may be configured to determine a model (e.g., a segmentation model) by training a preliminary model based on a plurality of training samples, more descriptions of which may be found elsewhere in the present disclosure (e.g., FIG. 9 and the descriptions thereof). In some embodiments, the model may be determined by a device external to the processing device 110. In some embodiments, the training module may be implemented by another processing device of the image analysis system 100 different from the processing device 110 illustrated in FIG. 4 and pre-stored in the storage device 150. The obtaining module 410 may obtain the segmentation model from the storage device 150 for use. Alternatively, the segmentation model may be pre-trained by a third party (e.g., a vendor for training and/or updating the segmentation module). The obtaining module 410 may obtain the segmentation model from the third party for use.

The modules in the processing device 110 may be connected to or communicate with each other via a wired connection or a wireless connection. The wired connection may include a metal cable, an optical cable, a hybrid cable, or the like, or any combination thereof. The wireless connection may include a Local Area Network (LAN), a Wide Area Network (WAN), a Bluetooth, a ZigBee, a Near Field Communication (NFC), or the like, or any combination thereof. In some embodiments, two or more of the modules of the processing device 110 may be combined as a single module, and any one of the modules of the processing device 110 may be divided into two or more units. In some embodiments, the processing device 110 may include one or more additional modules.

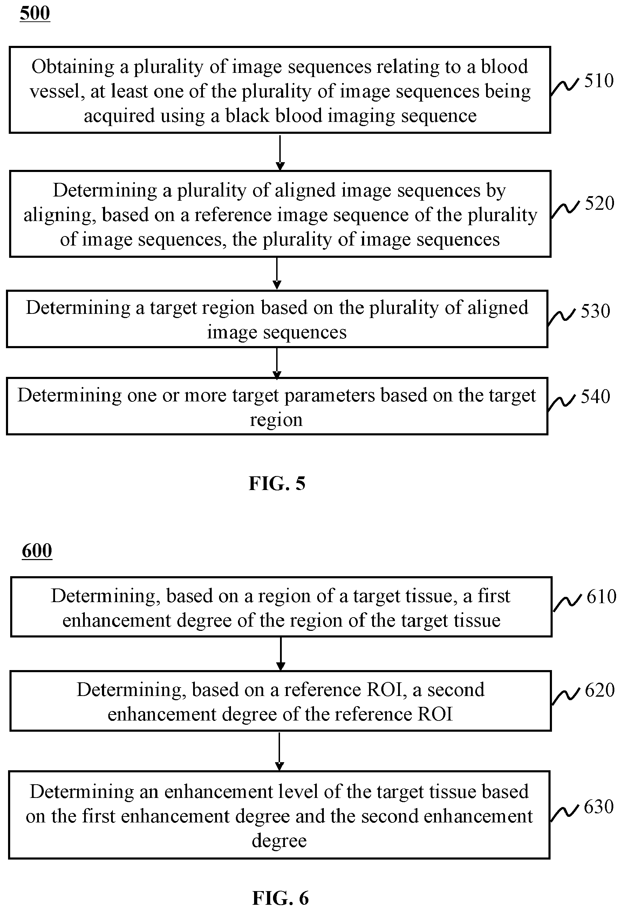

FIG. 5 is a flowchart illustrating an exemplary process for image analysis according to some embodiments of the present disclosure. In some embodiments, process 500 may be executed by the image analysis system 100. For example, the process 500 may be implemented as a set of instructions (e.g., an application) stored in a storage device (e.g., the storage device 150, the storage 220, and/or the storage 390). In some embodiments, the processing device 110 (e.g., the processor 210 of the computing device 200, the CPU 340 of the mobile device 300, and/or one or more modules illustrated in FIG. 4) may execute the set of instructions and may accordingly be directed to perform the process 500. The operations of the illustrated process presented below are intended to be illustrative. In some embodiments, the process 500 may be accomplished with one or more additional operations not described and/or without one or more of the operations discussed. Additionally, the order of the operations of process 500 illustrated in FIG. 5 and described below is not intended to be limiting.

In 510, the processing device 110 (e.g., the obtaining module 410) may obtain a plurality of image sequences relating to a blood vessel.

In some embodiments, the blood vessel may refer to a blood vessel of a subject. More descriptions of the subject may be found elsewhere in the present disclosure (e.g., FIG. 1 and relevant descriptions thereof). In some embodiments, the blood vessel may include a blood vessel of the brain, a blood vessel of the neck, a blood vessel of the heart, a blood vessel of a lung, etc. In some embodiments, the blood vessel may be of various types. For example, the blood vessel may include an arterial blood vessel, a venous blood vessel, and/or a capillary. In some embodiments, the blood vessel may include a lesion, such as, a plaque (e.g., a fibrous lipid), an ulceration, a thrombosis, an inflammation, an obstruction, a tumor, etc. A condition of the blood vessel (e.g., whether the blood vessel has a lesion) may be determined based on image(s) (e.g., the plurality of image sequences) relating to the blood vessel. As used herein, an image sequence relating to the blood vessel may refer to an image sequence that includes the blood vessel.

In some embodiments, an image sequence may refer to a series of images acquired in sequence. For example, the image sequence(s) may be a series of images relating to the blood vessel that are continuously acquired at different times and/or from different directions in sequence. In some embodiments, each of the plurality of image sequences may include one or more images to be processed. When an image sequence includes one image, the image sequence may be a 2D image. When an image sequence includes multiple images, the image sequence may be a 3D image. The counts (the numbers) of image(s) in different image sequences of the plurality of image sequences may be the same or different. For example, one image sequence of the plurality of image sequences may include one image to be processed, and another image sequence of the plurality of images may include three images to be processed. As another example, the plurality of image sequences may both include four images to be processed. Thus, the plurality of image sequences may include a plurality of images to be processed. In some embodiments, the contrasts of the plurality of image sequences may be the same or different.

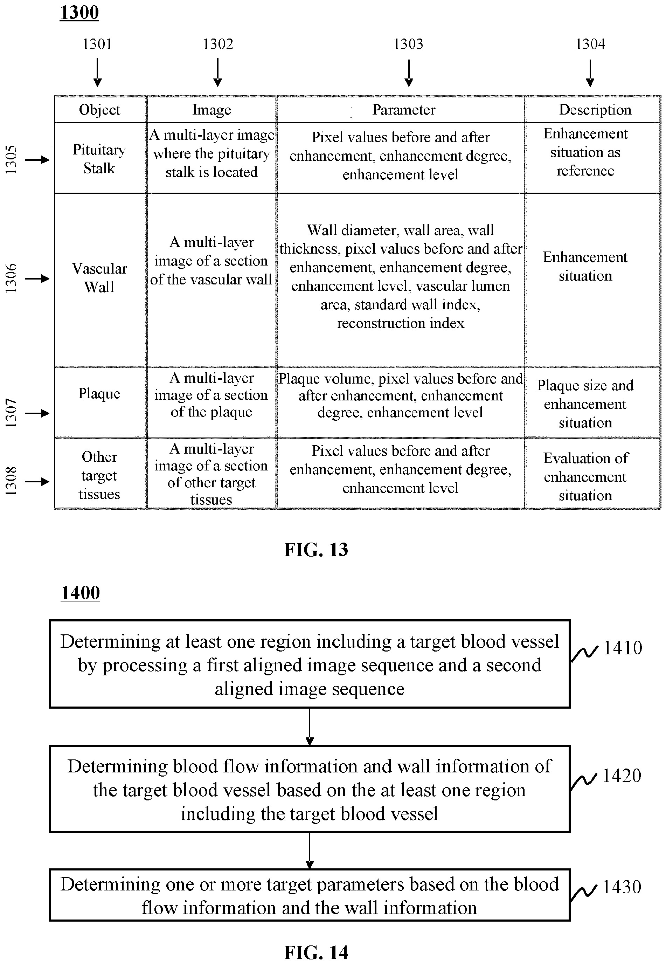

In some embodiments, the plurality of image sequences may be acquired using different types of imaging sequences. As used herein, an imaging sequence (e.g., MRI sequence) refers to a number of radio-frequency pulses (from an MRI device) and gradients that result (from protons in the subject) in a set of images with a particular appearance. The different types of imaging sequences may include a black blood imaging sequence, a bright blood imaging sequence, etc. A black blood imaging sequence may suppress blood flow signals so as to cause a lumen of the blood vessel to be black in an acquired image, thereby clearly displaying vascular wall information, plaque and/or aneurysm information, etc. A bright blood imaging sequence may cause the lumen of the blood vessel to be represented in high signals so as to improve a capability of displaying the vascular wall. Image sequences acquired using the black blood imaging sequence and the bright blood imaging sequence may be combined to represent related vascular information of the blood vessel more clearly. Exemplary black blood imaging sequences may include a T1 imaging sequence (e.g., a T 1-weighed imaging sequence), an enhanced T 1 imaging sequence, a T2 imaging sequence (e.g., a T2-weighted imaging sequence), a proton density imaging sequence (i.e., a proton-density-weighted imaging sequence), a fast spin echo (FSE) sequence, an inversion recovery (IR) sequence, a susceptibility weighted angiography (SWAN), or the like, or any combination thereof. Exemplary bright blood imaging sequences may include a time of flight (TOF) sequence, a phase contrast (PC) sequence, a fast imaging employing steady-state acquisition (FIESTA) sequence, an arterial spin labeling (ASL) sequence, a contrast-enhanced magnetic resonance angiography (CEMRA) sequence, etc. For example, at least one of the plurality of image sequences may be acquired using a black blood imaging sequence. In some embodiments, one of the plurality of image sequences relating to the blood vessel may be acquired using a time-resolved 3-dimensional magnetic resonance phase contrast imaging sequence (i.e., a 4D flow MRI sequence), more descriptions of which may be found elsewhere in the present disclosure (e.g., FIG. 14 and relevant descriptions thereof).

In some embodiments, the plurality of image sequences relating to the blood vessel may be obtained through an imaging device (e.g., the imaging device 140). For example, the plurality of image sequences relating to the blood vessel may be obtained through an MRI device, a CT device, a DSA device, an IVUS device, or the like, or any combination thereof. Merely by way of example, the imaging device 140 may collect image data of the subject or a portion thereof (e.g., the head, the neck, an arm, or the back, etc., of the subject). The imaging device 140 may transmit the image data of the subject to the processing device 110. The processing device 110 may receive the image data of the subject. Alternatively, the processing device 110 may directly obtain the image data of the subject from the imaging device 140. The image data relating to the subject may include vascular data, data of perivascular tissues, etc. The processing device 110 may extract the vascular data from the image data of the subject by, e.g., inputting the image data of the subject into an extraction model. The extraction model may include a trained model (e.g., a region-based convolutional network model, a fast region-based convolutional network model, a single shot multibox detector, etc.), more details of which may be found elsewhere in the present disclosure (e.g., FIG. 9 and descriptions thereof). The processing device 110 may perform an image reconstruction operation on the vascular data to generate one or more three dimensional (3D) images of the blood vessel. Exemplary image reconstruction algorithms may include a Fourier transform algorithm, a back projection algorithm (e.g., a convolution back projection algorithm, or a filtered back projection t algorithm), an iteration reconstruction algorithm, etc. The processing device 140 may perform a pre-processing operation on the one or more 3D images of the blood vessel to obtain the plurality of image sequences relating to the blood vessel. The pre-processing may include rotation, translation, correction, cutting, etc. In some embodiments, the processing device 140 may generate a plurality of image sequences relating to the subject based on the image data of the subject. The processing device 110 may determine the plurality of image sequences relating to the blood vessel based on the plurality of image sequences relating to the subject (e.g., by performing an extraction operation on the plurality of image sequences relating to the target tissue).

In some embodiments, the processing device 110 may obtain the plurality of image sequences relating to the blood vessel from one or more components (e.g., the terminal 130, the imaging device 140, and/or the storage device 150) of the image analysis system 100 or an external storage device via the network 120. For example, the plurality of image sequences relating to the blood vessel may be pre-stored in a storage device (e.g., the storage device 150, or the external storage device) for storage. The processing device 110 may obtain the plurality of image sequences relating to the blood vessel from the storage device. As another example, the processing device 110 may obtain the plurality of image sequences relating to the blood vessel from the imaging device 140 directly. The imaging device 140 may scan the subject or a portion thereof to obtain the plurality of image sequences relating to the blood vessel at different time points. In some embodiments, the imaging device 140 may adjust scanning parameters (e.g., a contrast, a resolution, a signal-to-noise ratio (SNR), etc.) to obtain the plurality of image sequences relating to the blood vessel during an examination of the subject or a portion thereof. The imaging device 140 may obtain the plurality of image sequences relating to the blood vessel generated using a same scanning parameter or different scanning parameters. The imaging device 140 may load the plurality of image sequences relating to the blood vessel into the image analysis system 100.

In 520, the processing device 110 (e.g., the aligning module 420) may determine a plurality of aligned image sequences by aligning the plurality of image sequences based on a reference image sequence of the plurality of image sequences

As used herein, the reference image sequence may refer to a standard image sequence for other image sequences among the plurality of image sequences. In some embodiments, the reference image sequence may include an image sequence that is acquired by a T1 imaging sequence among the plurality of image sequences. The T 1 imaging sequence may refer to a weighted sequence of longitudinal relaxation time (T1). The T1 imaging sequence may be a type of the black blood imaging sequence, and information of a sectional plane of the blood vessel may be acquired by the T1 imaging sequence. The T1 imaging sequence may also be referred to as a pre-drug imaging sequence. That is, the T1 imaging sequence may be performed before the subject is injected with a drug. As used herein, the drug may refer to or include contrast agent. For example, the contrast agent may be injected into the blood vessel, thereby achieving a better displaying performance of related information about the blood vessel.

In some embodiments, the processing device 110 may select the reference image sequence from the plurality of image sequences based on an image feature (e.g., clarity, resolution, etc.) of each of the plurality of image sequences. For example, the processing device 110 may obtain the clarity, resolution, etc., of each of the plurality of image sequences. The processing device 110 may select an image sequence with an optimal clarity, resolution, etc., among the plurality of image sequences as the reference image sequence. In some embodiments, the processing device 110 may directly designate one of the plurality of image sequences as the reference image sequence. For example, the processing device 110 may determine an image sequence acquired using the T1 imaging sequence among the plurality of image sequences as the reference image sequence.

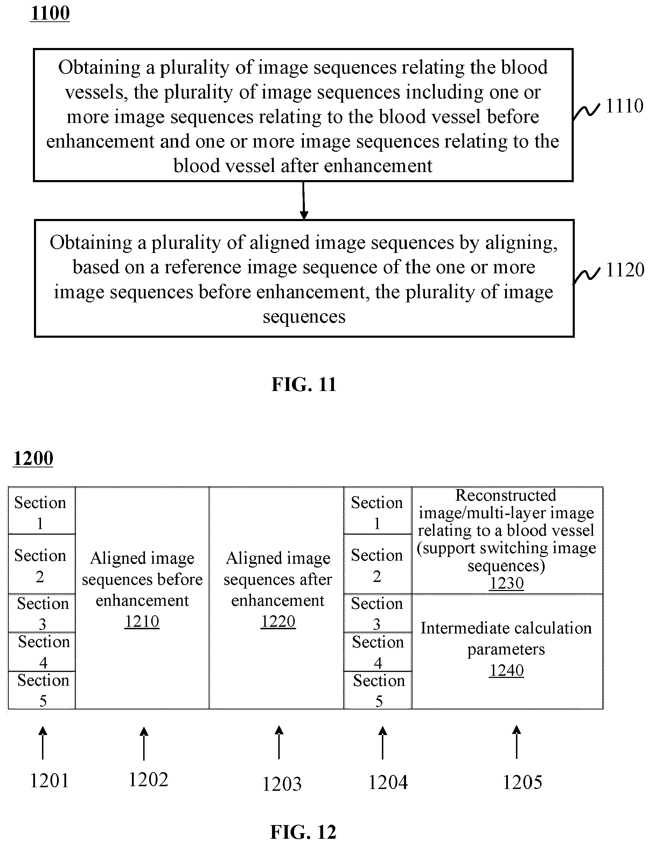

In some embodiments, since the plurality of image sequences may include images of the blood vessel acquired at different time points and/or using different imaging sequences, and/or the swallowing and/or movement of the subject may cause differences or offsets of the vascular morphology or vascular positions in the plurality of image sequences, the plurality of image sequences may need to be aligned with each other. The processing device 110 may determine the plurality of aligned image sequences by aligning, based on the reference image sequence, the plurality of image sequences through image registration. The image registration may include non-rigid registration, rigid registration (e.g., image rigid registration), affine registration, etc. For example, the blood vessels in the plurality of image sequences may be aligned based on the blood vessels in the reference image sequence, such that positions of the blood vessels in the plurality of image sequences may be aligned with the position of the blood vessel in the reference image sequence. More details about determining the plurality of aligned image sequences may be found elsewhere in the present disclosure (e.g., FIG. 11 and relevant descriptions thereof).

In some embodiments, according to the alignment of the plurality of image sequences, a plurality of images of the blood vessel corresponding to the same position may be viewed synchronously for performing quantitative or qualitative analysis, thereby determining comprehensive information of the blood vessel, which can improve the analysis accuracy of a plaque, an aneurysms, a stenosis plane, etc., in the blood vessel. In some embodiments, the plurality of aligned image sequences may be displayed according to a user instruction. For example, the processing device 110 may cause the plurality of aligned image sequences or a portion thereof to be displayed for comparison and viewing according to the user instruction. As another example, the processing device 110 may cause the plurality of aligned image sequences or a portion thereof to be displayed in different contrasts according to the user instruction. As a further example, the user may browse or switch the plurality of aligned image sequences or a portion thereof according to actual needs (e.g., an actual clinical need). In some embodiments, the processing device 110 may cause the plurality of aligned image sequences or a portion thereof to be displayed in adaptive layout based on, e.g., a count of one or more image sequences to be displayed.

In 530, the processing device 110 (e.g., the determining module 430) may determine a target region based on the plurality of aligned image sequences.

In some embodiments, the target region may include a region of a target tissue and/or a reference region of interest (ROI). In some embodiments, the region of the target tissue and the reference ROI may be independent regions in each of the plurality of aligned image sequences. In some embodiments, the region of the target tissue and the reference ROI may be adjacent regions in each of the plurality of aligned image sequences.

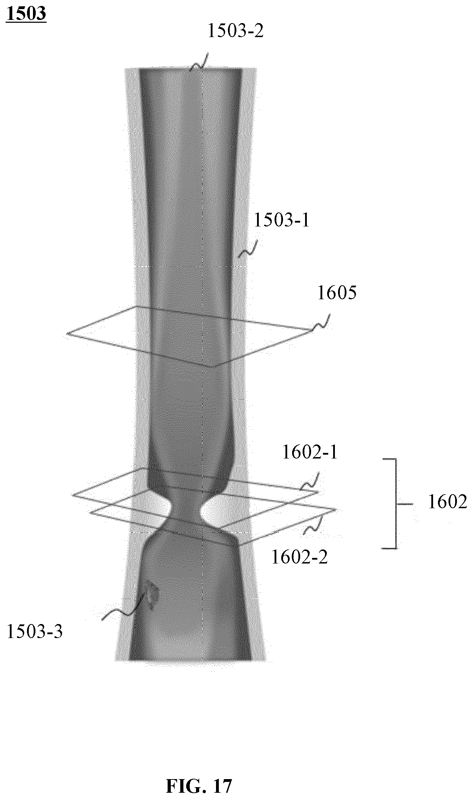

As used herein, the region of the target tissue may refer to a region where the target tissue is located or a region required to be processed (e.g., an ROI 1503-3 shown in FIG. 17) in each of the plurality of aligned image sequences. The target tissue may refer to any tissue of the subject. For example, the target tissue may include a head, a neck, arms, etc. In some embodiments, the region of the target tissue may include a region of a target blood vessel, and the target tissue may include a vascular wall, a plaque, an aneurysm, etc., of the target blood vessel. The target blood vessel may refer to an examined blood vessel (e.g., a part of the blood vessel described in FIG. 1). For example, the target blood vessel may include a part of a carotid artery vessel, a coronary vessel, a cerebral vessel, etc., according to the type of the blood vessel. In some embodiments, the target blood vessel (i.e., the part of the blood vessel) may include a plaque, a stenosis plane, an aneurysm, etc., of the blood vessel. As used herein, the reference ROI may refer to a standard region in each of the plurality of image sequences. In some embodiments, the reference ROI may include a pituitary stalk region, a muscle region, a muscle fiber region, a cerebrospinal fluid region, etc. In some embodiments, the reference ROI may be a region determined based on actual clinical needs.

In some embodiments, the processing device 110 may determine the target region (e.g., the region of the target tissue and/or the reference ROI) by identifying the target region in each of the plurality of aligned image sequences through a manual manner, a semi-automatic manner, an automatic manner (e.g., an artificial intelligence recognition), etc. For example, the target region may be determined through a delineation instruction, a delineation tool of the analysis system 100, and/or a delineation software, etc. More details of determining the target region may be found elsewhere in the present disclosure (e.g., FIG. 7 and FIG. 14 and descriptions thereof). As another example, the processing device 110 may identify/segment the target region from an aligned image sequence based on a deep learning model (e.g., a three-dimensional (3D) segmentation network model such as a V-Net model). An input of the 3D segmentation network model may include the aligned image sequence, etc., and an output of the 3D segmentation network model may include the target region of the aligned image sequence, etc. Alternatively, the processing device 110 may adjust the output of the 3D segmentation network model according to actual needs to obtain the target region of the aligned image sequence. For example, the output of the 3D segmentation model may include the target region, and the processing device 110 may zoom in or zoom out the target region according to actual needs of a user. For instance, the output of the 3D segmentation model may be an image with a size of 256*256, and the processing device 110 may adjust the image from the size of 256*256 to a size of 512*512 by zooming in the image. More details about training the 3D segmentation network model may be found elsewhere in the present disclosure (e.g., FIG. 9 and descriptions thereof). In some embodiments, the processing device 110 may transmit the target region among the plurality of aligned image sequences. For example, the processing device 110 may identify the target region in one aligned image sequence of the plurality of aligned image sequences. The processing device 110 may transmit the target region of the aligned image sequence to the rest of the plurality of the aligned image sequences. That is, the processing device 110 may determine the target region in another aligned image sequences based on the target region of the aligned image sequence (e.g., a range thereof and/or a location thereof in the aligned image sequence).

In some embodiments, the target region (e.g., the region of the target tissue and/or the reference ROI) may include a position of the target region, a range of the target region, a maximum section of the target region, etc. The maximum section of the target region may refer to a plane with a maximum sectional area in the target region. The processing device 110 may determine the position of the target region, the range of the target region, the maximum section of the target region, etc., based on the target region segmented or identified from the aligned image sequence.

It should be noted that the region of the target tissue and the region of the reference ROI may refer to a corresponding region in an image. The region of the target tissue and the region of the reference ROI may also be referred as the "region," "image of a region," or "region in an image" in the present disclosure.

In 540, the processing device 110 (e.g., the determining module 430) may determine one or more target parameters based on the target region. The one or more target parameters may refer to parameters relating to the target tissue (e.g., vascular information of interest of the target blood vessel) in the target region, which can represent a condition of the target tissue in the target region.

In some embodiments, the one or more target parameters may include an enhancement level of the target tissue (e.g., the target blood vessel). The enhancement level of the target tissue may refer to a parameter used for qualitative evaluation of the target tissue. For example, a greater enhancement level of the plaque may indicate the instability of the plaque. The plaque may reflect a level of inflammatory activity. Different enhancement levels of the plaque may be associated with different acute vascular events. A significant enhancement level of the plaque may suggest an instability of the plaque. The instability of the plaque may be prone to the acute cardiovascular event. Thus, the enhancement level of the plaque may be an important indicator in an assessment of an intracranial plaque and the most important parameter in the qualitative assessment of the plaque.

In some embodiments, the one or more target parameters may include a kinetic parameter of blood flow, a morphological parameter, etc., of the target blood vessel. The kinetic parameter of blood flow may include a parameter of blood flow in the lumen of the target blood vessel, etc. The morphological parameter of the target blood vessel may include a parameter of vascular wall of the target blood vessel, etc. For example, , the one or more target parameters may include at least one of a flow quantity of the target blood vessel, a flow rate of a target blood vessel, a wall thickness of a target blood vessel, a wall shear force of a target blood vessel, a volume of the target blood vessel, data relating to the maximum section of the target blood vessel, or the like, or any combination thereof. The data relating to the maximum section of the target blood vessel may include a flow quantity, a flow rate, etc., relating to the maximum section of the target blood vessel.

The flow quantity of the target blood vessel may refer to a parameter of blood flow, such as an amount of blood passing through a plane in a unit time. In some embodiments, the flow quantity of the target blood vessel may include a bidirectional blood flow (e.g., forward blood flow and backward blood flow), a stroke volume, an average flow, etc. The processing device 110 may determine a blood flowing status of the target blood vessel based on the flow quantity of the target blood vessel. The flow quantity of the target blood vessel may be a type of kinetic parameters of blood flow.

The flow rate of the target blood vessel may refer to a velocity parameter of blood flow, such as a displacement of blood per unit time. In some embodiments, the flow rate of the target blood vessel may include an average flow rate, a peak flow rate, etc. The flow rate of the target blood vessel may indicate a condition that the blood passing through a plane. The flow rate of the target blood vessel may be a type of kinetic parameters of blood flow.

The wall thickness of the target blood vessel may refer to a parameter indicating the thickness of a vascular wall, such as a thickness of the vascular wall, a standard index of the vascular wall, etc. The wall thickness of the target blood vessel may be a type of kinetic parameters of blood flow.

The wall shear force of the target blood vessel may refer to a viscous shear force of flowing blood acting tangentially on the vascular wall. The wall shear force of the target blood vessel may include a maximum wall shear force of the target blood vessel, an average wall shear force of the target blood vessel, etc. The wall shear force of the target blood vessel may be a type of kinetic parameters of blood flow.

In some embodiments, the processing device 110 may determine the one or more target parameters by performing an image extraction (or segmentation), an image reconstruction, etc., on the target region (e.g., the target tissue such as the target blood vessel). More details of determining the one or more target parameters may be found elsewhere in the present disclosure (e.g., FIG. 6, FIG. 10, FIG. 14, and FIG. 15 and descriptions thereof).

It should be noted that the above description regarding the process 500 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 500 may be omitted, and/or one or more additional operations may be added.

FIG. 6 is a schematic diagram illustrating an exemplary process for a determination of an enhancement level of a target tissue according to some embodiments of the present disclosure. In some embodiments, a process 600 may be executed by the image analysis system 100. For example, the process 600 may be implemented as a set of instructions (e.g., an application) stored in a storage device (e.g., the storage device 150, the storage 220, and/or the storage 390). In some embodiments, the processing device 110 (e.g., the processor 210 of the computing device 200, the CPU 340 of the mobile device 300, and/or one or more modules illustrated in FIG. 4) may execute the set of instructions and may accordingly be directed to perform the process 600. The process 600 may include following operations. In some embodiments, a portion of operation 540 in FIG. 5 may be achieved by the process 600.

In 610, the processing device 110 (e.g., the determining module 430) may determine a first enhancement degree of the region of the target tissue based on the region of the target tissue.

As used herein, an enhancement degree may refer to a contrast degree. The enhanced degree of a region may refer to a contrast degree of the region in an image. In some embodiments, the processing device 110 may perform an image processing on a region to determine the enhancement degree of the region. For example, the image processing may include at least one of an arithmetic processing, an analysis processing, a comparing processing, an image sharpening, etc.

In some embodiments, the processing device 110 may determine the region of the target tissue by processing the plurality of aligned image sequences based on the position of the target region, the range of the target region, the maximum section of the target region, etc.

In some embodiments, the processing device 110 may determine the first enhancement degree of the region of the target tissue by performing an image processing on the region of the target tissue. In some embodiments, the region of target tissue may include a region of the target tissue before enhancement and a region of the target tissue after enhancement. For example, the region of the target tissue before enhancement may refer to a region of the target tissue before an infusion/injection of the drug, and the region of the target tissue after enhancement may refer to a region of the target tissue after the infusion/injection of the drug. The region of the target tissue after enhancement may have distinct characteristics relative to the region of the target tissue before enhancement, so that the region of the target tissue may be identified easily during subsequent image processing.

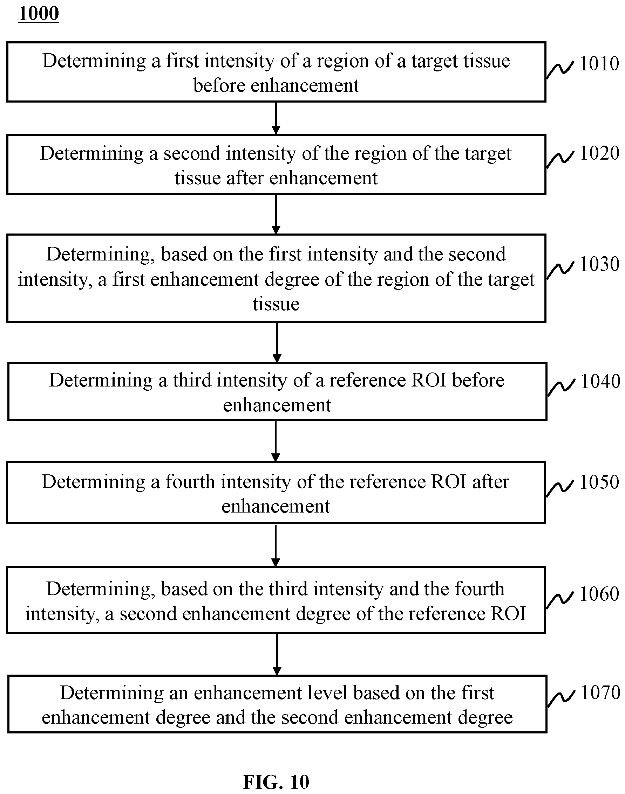

In some embodiments, the processing device 110 may determine the first enhancement degree of the region of the target tissue based on the region of the target tissue before enhancement and the region of the target tissue after enhancement. More details about determining the first enhancement degree of the region of the target tissue may be found elsewhere in the present disclosure (e.g., FIG. 10 and related descriptions thereof).

In 620, the processing device 110 (e.g., the determining module 430) may determine a second enhancement degree of the reference ROI based on the reference ROI.

In some embodiments, the processing device 110 may determine the second enhancement degree of the reference ROI by performing an image processing on the reference ROI. Similar to the region of the target tissue, the reference ROI may include a reference ROI before enhancement and a reference ROI after enhancement. For example, the reference ROI before enhancement may refer to a reference ROI before an infusion/injection of the drug, and the reference ROI after enhancement may refer to a reference ROI after the infusion/injection of the drug.

In some embodiments, the processing device 110 may determine the second enhancement degree of the reference ROI based on the reference ROI before enhancement and the reference ROI after enhancement. More details about determining the second enhancement degree of the reference ROI may be found elsewhere in the present disclosure (e.g., FIG. 10 and related descriptions thereof).

In 630, the processing device 110 (e.g., the determining module 430) may determine the enhancement level of the target tissue based on the first enhancement degree and the second enhancement degree.

In some embodiments, the processing device 110 may determine the enhancement level of the target tissue by performing the arithmetic operation on the first enhancement degree and the second enhancement degree. For example, the arithmetic operation may include a comparing operation, an adding operation, a subtraction operation, a multiplication operation, a dividing operation, a logarithm operation, etc. In some embodiments, the enhancement level of the target tissue may be represented as a quantitative value of the enhancement level. More details about determining the enhancement level of the target tissue may be found elsewhere in the present disclosure (e.g., FIG. 10 and related descriptions thereof).

It should be noted that the above description regarding the process 600 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 600 may be omitted, and/or one or more additional operations may be added.

FIG. 7 is a schematic diagram illustrating an exemplary process for a determination of a target tissue according to some embodiments of the present disclosure. In some embodiments, a process 700 may be executed by the image analysis system 100. For example, the process 700 may be implemented as a set of instructions (e.g., an application) stored in a storage device (e.g., the storage device 150, the storage 220, and/or the storage 390). In some embodiments, the processing device 110 (e.g., the processor 210 of the computing device 200, the CPU 340 of the mobile device 300, and/or one or more modules illustrated in FIG. 4) may execute the set of instructions and may accordingly be directed to perform the process 700. The process 700 may include following operations. In some embodiments, at least a portion of operation 530 (e.g., the determination of the target tissue such as a plaque of the blood vessel) in FIG. 5 may be achieved by the process 700.

In 710, the processing device 110 (e.g., the determining module 430) may determine a vascular wall by identifying the vascular wall from each of a plurality of aligned image sequences (e.g., the plurality of aligned image sequences in operation 520).

The blood vessel may include the lumen and the vascular wall. The lumen may refer to a pipeline used for blood flow. The vascular wall may refer to a tissue structure wrapping the lumen. The vascular wall may include an inner contour of the blood vessel and an outer contour of the blood vessel. The inner contour of the blood vessel may be the lumen.

In some embodiments, the processing device 110 may input the plurality of aligned image sequences into a wall recognition model, and the wall recognition model may determine the vascular wall by identifying the vascular wall from the plurality of aligned image sequences. In some embodiments, the wall recognition model may include a trained model. For example, the wall recognition model may be a region-based convolutional network model, a fast region-based convolutional network model, a single shot multibox detector, etc. More details of a trained model may be found elsewhere in the present disclosure (e.g., FIG. 9 and descriptions thereof).

In some embodiments, the processing device 110 may extract and/or identify the vascular wall from each of the plurality of aligned image sequences based on image reconstruction, such as a curved planar reconstruction (CPR), etc. For example, the processing device 110 may extract and/or identify the vascular wall by reconstructing a CPR image of the blood vessel in each of the plurality of aligned image sequences, and the processing device 110 may identify the lumen and the vascular wall from the CPR image of the blood vessel.

In some embodiments, the processing device 110 may determine the vascular wall through the delineation instruction, the delineation tool of the analysis system 100, and/or the delineation software (e.g., ITK-SNAP), etc.

In 720, the processing device 110 (e.g., the determining module 430) may determine the plaque by identifying the plaque from the vascular wall.

In some embodiments, the processing device 110 may input an image of the vascular wall into a plaque recognition model, and the plaque recognition model may determine the plaque by identifying the plaque from the vascular wall. In some embodiments, the plaque recognition model may include a trained model. For example, the plaque recognition model may be a region-based convolutional network model, a fast region-based convolutional network model, a single shot multibox detector, etc. More details of the trained model may be found elsewhere in the present disclosure (e.g., FIG. 9 and descriptions thereof).

In some embodiments, the processing device 110 may determine the plaque through the delineation instruction, the delineation tool of the analysis system 100, and/or the delineation software (e.g., ITK-SNAP), etc.

It should be noted that the above description regarding the process 700 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 700 may be omitted, and/or one or more additional operations may be added.

FIG. 8 is an exemplary flowchart illustrating an exemplary process for a determination of a reference ROI according to some embodiments of the present disclosure. In some embodiments, a process 800 may be executed by the image analysis system 100. For example, the process 800 may be implemented as a set of instructions (e.g., an application) stored in a storage device (e.g., the storage device 150, the storage 220, and/or the storage 390). In some embodiments, the processing device 110 (e.g., the processor 210 of the computing device 200, the CPU 340 of the mobile device 300, and/or one or more modules illustrated in FIG. 4) may execute the set of instructions and may accordingly be directed to perform the process 800. The process 800 may include following operations. In some embodiments, at least a portion of operation 530 in FIG. 5 may be achieved by the process 800.

In some embodiments, as shown in FIG. 8, the processing device 110 may determine the reference ROI based on a delineation instruction. The delineation instruction may be determined or generated from a user terminal (e.g., the terminal 130). For example, the delineation instruction may be generated by a user through a mouse, a keyboard, a voice, etc., of the terminal 130.

In 810, the processing device 110 (e.g., the obtaining module 410) may obtain the delineation instruction. In some embodiments, the delineation instruction may include position information about the reference ROI. Merely by way of example, the user may open a delineation tool, a delineation software (e.g., ITK-SNAP), etc., to set the reference ROI by inputting and/or selecting one or more setting items/parameters based on actual needs. The delineation instruction may be generated based on the setting items/parameters selected by the user and used for delineating a counter of the reference ROI in an image. The processing device 110 may obtain the delineation instruction. The one or more setting items/parameters may at least include position information about the reference ROI.

In 820, the processing device 110 (e.g., the determining module 430) may determine the reference ROI in each of the aligned image sequence(s) based on the position information about the reference ROI.

In some embodiments, the processing device 110 may extract the reference ROI in each of the aligned image sequence(s) based on the position information about the reference ROI and remove/filter other region except for the reference ROI.

It should be noted that the above description regarding the process 800 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 800 may be omitted, and/or one or more additional operations may be added.

In some embodiments, the processing device 110 may determine the reference ROI based on a template image. The template image may include a preset image corresponding to the reference ROI, an image of a preset tissue with a prominent tissue feature before and after the drug infusion/injection, etc. In some embodiments, a size of the template image may be (substantially) equal to a size of an image in the aligned image sequence(s) In some embodiments, the template image may include the reference ROI (or the preset tissue) and/or a background image. For example, the template image may only include the reference ROI or the preset tissue. As used herein, the background image may refer to an invalid image that is used to fill a part of the template image except for the reference ROI or the preset tissue, so that the size of the template image may be (substantially) equal to the size of an image in the aligned image sequence(s). The preset tissue with a prominent feature before and after the drug infusion/injection may include the smooth muscle, the sarcoplasmic, the nerve fibers, the pituitary stalk, etc. In some embodiments, the template image may be a 2D image or a 3D image corresponding to a type (i.e., a 2D image or a 3D image) of the aligned image sequence(s)

In some embodiments, the processing device 110 may perform the image registration on each of the plurality of aligned image sequences and the template image to determine the reference ROI. As used herein, the image registration may refer to mapping the preset tissue in the template to an aligned image sequence to determine the reference ROI. Taking the preset tissue of the pituitary stalk as an example, the pituitary stalk may be on the vascular wall of the blood vessel, and a tissue structure of the vascular wall may be similar to a tissue structure of the pituitary stalk. The processing device 110 may extract or segment the pituitary stalk from an aligned image sequence and determine the template image based on a region of the pituitary stalk in the aligned image sequence. The processing device 110 may further register the aligned image sequence and the template image to determine the reference ROI in the aligned image sequence. Alternatively, the processing device 110 may determine an image including a standard pituitary stalk as the template image. The processing device 110 may register the aligned image sequence and the template image to determine the reference ROI.

In some embodiments, the processing device 110 may input each of the plurality of aligned image sequences into a segmentation model to determine the reference ROI in the aligned image sequence. The segmentation model may include a trained model. For example, the segmentation model may include a fully convolutional network model, a fast convolutional network model, a fast regional-based convolutional network model, a mask regional-based convolutional network model, or the like, or any combination thereof. The training of the segmentation model may be described in FIG. 9.

FIG. 9 is an exemplary flowchart illustrating an exemplary training process of a segmentation model according to some embodiments of the present disclosure. In some embodiments, a process 900 may be performed by a training device, such as the processing device 110, a different processing device of the image analysis system 100, or a processing device external to the image analysis system 100 (e.g., a processing device of a vendor that generates, provides, maintains, and/or updates the segmentation model). For example, the process 900 may be implemented as a set of instructions (e.g., an application) stored in a storage device (e.g., the storage device 150, the storage 220, and/or the storage 390). In some embodiments, the processing device 110 (e.g., the processor 210 of the computing device 200, the CPU 340 of the mobile device 300, and/or one or more modules illustrated in FIG. 4) may execute the set of instructions and may accordingly be directed to perform the process 900. The operations of the illustrated process presented below are intended to be illustrative. In some embodiments, the process 900 may be accomplished with one or more additional operations not described and/or without one or more of the operations discussed. Additionally, the order of the operations of process 900 illustrated in FIG. 9 and described below is not intended to be limiting. Alternatively, the process 900 may be implemented by a processing device of a third party (e.g., n vendor) that provides the segmentation model.

In 910, the processing device 110 may obtain a plurality of training samples and a plurality of labels corresponding to the training samples.

In some embodiments, each of the training samples may include a sample image sequence and a label. The label corresponding to the sample image sequence may include a reference ROI. The sample image sequence may be an image sequence of a sample subject or a portion thereof (e.g., an image sequence of a blood vessel of the sample subject).

In some embodiments, at least a portion of a label of a training sample, e.g., a reference ROI, may be historical data obtained by scanning the sample subject using an imaging device (e.g., the imaging device 140). In some embodiments, at least a portion of a label of a training sample, e.g., a reference ROI, may be determined manually or semi-automatically. For example, a reference ROI may be determined based on an outline of a sample tissue of the sample subject represented in the sample image sequence by manual annotation or other manners.

Descriptions regarding the image sequence(s), the aligned image sequence(s), and the reference ROI elsewhere in the present disclosure (e.g., FIG. 5, FIG. 6, FIG. 8, and related descriptions thereof) are applicable to the sample image sequence(s) and the reference ROI, respectively, and not repeated herein.

In 920, the processing device 110 may generate the segmentation model by training a preliminary segmentation model based on the plurality of training samples and the plurality of labels.

The preliminary segmentation model may include multiple model parameters each of which is assigned an initial value. During the training, the values of the multiple model parameters may be updated iteratively. For instance, in one iteration, at least a portion of a training sample (e.g., the sample image sequence) may be input into the preliminary segmentation model or an intermediate segmentation model (that is partially training using at least one training sample set) obtained in a prior iteration (e.g., an iteration immediately preceding the current iteration) and a prediction result may be determined. The prediction result may include a predicted reference ROI determined by the preliminary or intermediate segmentation model. The prediction result may be compared with the label of the training sample.

The training processing device may adjust the values of model parameters of the preliminary or intermediate segmentation model based on the comparison of the prediction result with the label of the training sample. For instance, the training processing device may adjust the values of model parameters of the preliminary or intermediate segmentation model based on the comparison of the prediction result with the label of the training sample to reduce a difference between the prediction result and the label of the training sample. Merely by way of example, the training processing device may obtain a loss function that relates to the prediction result and the label of a training sample. The loss function may assess a difference between the prediction result and the label. A value of the loss function may be reduced or minimized (e.g., lower than a threshold) by iteratively adjusting the values of the model parameters. In some embodiments, the loss function may include a mean squared error loss function, a binary classification cross entropy loss function, a sparse binary classification cross entropy loss function, etc., which is not limited herein.

In some embodiments, for each of the training samples, a label corresponding to a sample image sequence may also include a region (e.g., the vascular wall, the plaque, etc.) of a target tissue. The training process mentioned above may also be applicable to the training samples, each of which includes the label of the region of the target tissue, and not be repeated herein.

It should be noted that the training samples may be selected based on one or more desired functions of a trained model. For instance, the trained model may be the extraction model as illustrated in FIG. 5 (operation 510 of the process 500), the recognition model as illustrated in FIG. 7 (operations 710 and 720 of the process 700), the 3D segmentation network model as illustrated in FIG. 5 (operation 530 of the process 500), the segmentation model as illustrated in FIG. 8, etc. The following descriptions are provided based on the determination of the segmentation model for illustration purposes and not intended to be limiting. It should be understood that other models described herein may be trained based on the process 900 using suitable training samples.

It should be noted that the above description regarding the process 900 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 900 may be omitted, and/or one or more additional operations may be added.

FIG. 10 is a flowchart illustrating an exemplary process for determining an enhancement level according to some embodiments of the present disclosure. In some embodiments, a process 1000 may be executed by the image analysis system 100. For example, the process 1000 may be implemented as a set of instructions (e.g., an application) stored in a storage device (e.g., the storage device 150, the storage 220, and/or the storage 390). In some embodiments, the processing device 110 (e.g., the processor 210 of the computing device 200, the CPU 340 of the mobile device 300, and/or one or more modules illustrated in FIG. 4) may execute the set of instructions and may accordingly be directed to perform the process 1000. The process 1000 may include following operations. In some embodiments, operations 610 and/or 620 in FIG. 6 may be achieved by the process 1000.

In 1010, the processing device 110 (e.g., the determining module 430) may determine a first intensity of the region of the target tissue before enhancement.

As used herein, an intensity of the region of the target tissue may refer to a characteristic value of pixels in the region of the target tissue. The characteristic value may include a pixel characteristic value of the pixels in the region of the target tissue, a signal intensity of the pixels in the region of the target tissue, or the like, or any combination thereof. For example, the pixel characteristic value may include at least one of a maximum pixel value of the pixels in the region of the target tissue, a minimum pixel value of the pixels in the region of the target tissue, an average pixel value of the pixels in the region of the target tissue, a variance of pixel values of the pixels in the region of the target tissue, a standard deviation of pixel values of the pixels in the region of the target tissue, etc. As another example, the signal intensity characteristic value may include at least one of a maximum signal intensity of the pixels in the region of the target tissue, a minimum signal intensity of the pixels in the region of the target tissue, an average signal intensity of the pixels in the region of the target tissue, a variance of signal intensities of the pixels in the region of the target tissue, a standard deviation of signal intensities of the pixels in the region of the target tissue, etc.

In some embodiments, the first intensity of the region of the target tissue may refer to an intensity of the region of the target tissue before enhancement. For example, the first intensity may be the intensity of the region of the target tissue before the target tissue is infused/injected with the drug. In some embodiments, the processing device 110 may determine the first intensity of the region of the target tissue before enhancement by performing the arithmetic operation on the characteristic value of the pixels in the region of the target tissue before enhancement. The arithmetic operation may include a comparing operation, an adding operation, a subtraction operation, a multiplication operation, a dividing operation, a logarithm operation, etc.

In 1020, the processing device 110 (e.g., the determining module 430) may determine a second intensity of the region of the target tissue after enhancement.

In some embodiments, the second intensity of the region of the target tissue may refer to an intensity of the region of the target tissue after enhancement. For example, the second intensity may be the intensity of the region of the target tissue after the target tissue is infused/injected with the drug. Similar to the determination of the first intensity, the processing device 110 may determine the second intensity of the region of the target tissue after enhancement by performing the arithmetic operation on the characteristic value of the pixels in the region of the target tissue determined after enhancement.

In 1030, the processing device 110 (e.g., the determining module 430) may determine the first enhancement degree of the region of the target tissue based on the first intensity and the second intensity.

In some embodiments, the processing device 110 may determine the first enhancement degree of the region of the target tissue by processing the first intensity and the second intensity. For example, the processing device 110 may determine the first enhancement degree of the region of the target tissue by dividing the second intensity by the first intensity.

In 1040, the processing device 110 (e.g., the determining module 430) may determine a third intensity of the reference ROI before enhancement.

Descriptions regarding an intensity of the region of the target tissue, the first intensity of the region of the target tissue before enhancement, and the determination of the first intensity elsewhere in the present disclosure (e.g., relevant descriptions of FIG. 6, FIG. 10, and the operations 610 and 1030) may be applicable to an intensity of the reference ROI, the third intensity of the reference ROI before enhancement, and a determination of the third intensity, respectively, and not repeated herein.

In 1050, the processing device 110 (e.g., the determining module 430) may determine a fourth intensity of the reference ROI after enhancement.

Descriptions regarding the second intensity of the region of the target tissue after enhancement and the determination of the second intensity elsewhere in the present disclosure (e.g., relevant descriptions of FIG. 6, FIG. 10, and the operations 620 and 1060) may be applicable to the fourth intensity of the reference ROI after enhancement and a determination of the fourth intensity, respectively, and not repeated herein.

In 1060, the processing device (e.g., the determining module 430) may determine the second enhancement degree of the reference ROI based on the third intensity and the fourth intensity.

In some embodiments, the processing device 110 may determine the second enhancement degree of the reference ROI by processing the third intensity and the fourth intensity. For example, the processing device 110 may determine the second enhancement degree of the reference ROI by dividing the fourth intensity by the third intensity.

In operation 1070, the processing device 110 (e.g., the determining module 430) may determine the enhancement level of the target tissue based on the first enhancement degree and the second enhancement degree. For instance, the processing device 110 may determine the enhancement level of the target tissue by dividing the first enhancement degree by the second enhancement degree.

In some embodiments, the enhancement level of the target tissue may include a confidence level. The confidence level may refer to a credibility degree of the enhancement level. The confidence level may be represented as a real number, a percentage, a rank, etc. For example, the confidence level may be a real number in a range of 0 to 1.

In some embodiments, the processing device 110 may determine the confidence level of the enhancement level based on a value of the enhancement level deviating from a preset range. The value of the enhancement level deviating from the preset range may refer to a difference between the enhancement level and an evaluation value of the preset range. The evaluation value of the preset range may include a minimum value of the preset range, a maximum value of the preset range, an average value of the preset range, a mean value of the preset range, etc. The preset range may be determined based on a manual setting or a system setting. If the value of the enhancement level deviating from the preset range exceeds a threshold (e.g., a percentage threshold such as 5%, 10%, 20%, etc.), the target tissue may have a lesion or the enhancement level of the target tissue may be inaccurate. In some embodiments, in response to determining that the value of the enhancement level deviating from the preset range exceeds the threshold, the processing device 110 may determine whether the target tissue has the lesion or the enhancement level of the target tissue is inaccurate based on a decision model (e.g., a support vector machine (SVM) model). For example, the processing device 110 may input the value of the enhancement level deviating from the preset range into the decision model. If the decision model outputs a positive value (e.g., "1"), the processing device 110 may determine that the target tissue has the lesion. If the decision model outputs a negative value (e.g., "-1"), the processing device 110 may determine that the enhancement level of the target tissue is inaccurate, and the confidence level of the enhancement level is low, so that operations 1010-1070 may be adjusted and re-performed herein. In some embodiments, in response to determining that the value of the enhancement level deviating from the preset range does not exceed the threshold, the processing device 110 may determine that the confidence level of the enhancement level is high, and operations 1010-1070 may be terminated.

In some embodiments, the processing device 110 may determine the confidence level of the enhancement level based on the value of the enhancement level deviating from a preset range and a segmentation confidence level of the reference ROI. The segmentation confidence level of the reference ROI may be a preset value determined by using a segmentation algorithm. The segmentation algorithm may include an edge-based segmentation algorithm, a regional-based segmentation algorithm, a graph-based segmentation algorithm, etc. For example, the processing device 110 may determine the confidence level of the enhancement level by multiplying the value of the enhancement level deviating from a preset range and the segmentation confidence level of the reference ROI.

In some embodiments, the processing device 110 may determine the confidence level of the enhancement level based on an area of the region of the target tissue and an area of the reference ROI. The area of the region of the target tissue and/or the area of the reference ROI may refer to a count of the pixels in the region of the target tissue and/or the reference ROI that are used to determine the enhancement level of the target tissue. For example, the processing device 110 may determine a coefficient of the confidence level by dividing the area of the reference ROI by the area of the region of the target tissue, and determine the confidence level of the enhancement level by multiplying the coefficient of the confidence level, the value of the enhancement level deviating from a preset range, and the segmentation confidence level of the reference ROI.

It should be noted that the above description regarding the process 1000 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 1000 may be omitted, and/or one or more additional operations may be added.

FIG. 11 is a flowchart illustrating an exemplary process for a determination of a plurality of aligned image sequences according to some embodiments of the present disclosure. In some embodiments, a process 1100 may be executed by the image analysis system 100. For example, the process 1100 may be implemented as a set of instructions (e.g., an application) stored in a storage device (e.g., the storage device 150, the storage 220, and/or the storage 390). In some embodiments, the processing device 110 (e.g., the processor 210 of the computing device 200, the CPU 340 of the mobile device 300, and/or one or more modules illustrated in FIG. 4) may execute the set of instructions and may accordingly be directed to perform the process 1100. The process 1100 may include following operations. In some embodiments, at least a portion of operation 520 in FIG. 5 may be achieved by the process 1100.

In 1110, the processing device 110 (e.g., the obtaining module 410) may obtain a plurality of image sequences relating to the blood vessel. The plurality of image sequences may include one or more image sequences relating to the blood vessel before enhancement and one or more image sequences relating to the blood vessel after enhancement.

In some embodiments, the one or more image sequences relating to the blood vessel before enhancement may include image sequence(s) relating to a black blood vessel and/or a bright blood vessel before enhancement. In some embodiments, the one or more image sequences relating to the blood vessel after enhancement may include image sequence(s) relating to the black blood vessel and/or the bright blood vessel after enhancement. For example, if the plurality of image sequences includes an image sequence relating to a black blood vessel before enhancement, the plurality of image sequences may also include an image sequence relating to the black blood vessel after enhancement.

In some embodiments, the imaging device 140 may acquire first data of the subject or a portion thereof before the subject is infused with drugs, and transmit the first data to the processing device 110. The processing device 110 may extract first vascular data from the first data to, and determine one or more first images of the blood vessel (e.g., a 3D image) by performing the image reconstruction on the first vascular data. The processing device 110 may process the one or more first images of the blood vessel to obtain an image sequence relating to the blood vessel before 2D enhancement. For example, the processing device 110 may determine a first vascular centerline by performing a skeletonizing operation on the one or more first images of the blood vessel (e.g., the 3D image). As used herein, the skeletonizing operation may refer to an operation that removes a boundary of an image relating to a blood vessel using a morphological etching operation to obtain a centerline of the blood vessel in the image. The processing device 110 may determine a 2D images corresponding to vascular cross-sections of the blood vessel (i.e., an image sequence relating to the blood vessel before 2D enhancement) by cutting, based on the one or more first images of the blood vessel, the one or more first images (i.e., the 3D image) of the blood vessel.

In some embodiments, the imaging device 140 may acquire second data of the subject or a portion thereof after the subject is infused with drugs, and transmit the second data to the processing device 110. The processing device 110 may extract second vascular data from the second data, and determine one or more second images (e.g., a 3D image) of the blood vessel by performing the image reconstruction on the second vascular data. The processing device 110 may process the one or more second images of the blood vessel to obtain an image sequence relating to the blood vessel after 2D enhancement. For example, the processing device 110 may determine a second vascular centerline by performing a skeletonizing operation on the one or more second images (e.g., the 2D image) of the blood vessel. The processing device 110 may determine 2D images corresponding to vascular cross-sections of the blood vessel (i.e., an image sequence relating to the blood vessel after 2D enhancement) by cutting, based on the one or more second images of the blood vessel, the one or more second images (i.e., the 2D image) of the blood vessel.

In 1120, the processing device 110 (e.g., the obtaining module 410) may obtain the plurality of aligned image sequences by aligning, based on a reference image sequence of the one or more image sequences before enhancement, the plurality of image sequences.

Since a motion/displacement of the subject may occur when vascular data of the target tissue is collected, the vascular data may be different due to different positions of the subject per collection. The processing device 110 may perform motion correction on the one or more image sequences relating to the blood vessel before enhancement and the one or more image sequences relating to the blood vessel after enhancement for correcting motion artifacts to obtain the plurality of aligned image sequences (e.g., images of the blood vessel corresponding to the same position of the subject).

In some embodiments, for the motion correction, the processing device 110 may determine the reference image sequence from the one or more image sequences before enhancement. The processing device 110 may perform an image registration on the one or more image sequences before enhancement and the one or more image sequences after enhancement based on the reference image sequence to obtain the plurality aligned image sequences.

It should be noted that the above description regarding the process 1100 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 1100 may be omitted, and/or one or more additional operations may be added.

FIG. 12 is a schematic diagram illustrating an exemplary layout of a plurality of aligned image sequences according to some embodiments of the present disclosure. The layout of the plurality of aligned image sequences may be in a form of a table 1200. It should be noted that the table 1200 illustrated in FIG. 12 may be an exemplary layout of the plurality of aligned image sequences, which is not intended to limit the scope of the present disclosure. The plurality of aligned image sequences may include aligned image sequences 1210 before enhancement and aligned image sequences 1220 after enhancement.

As shown in FIG. 12, "Section 1," "Section 2," "Section 3," "Section 4," and "Section 5" in a first column 1201 of the table 1200 may refer to labels of the aligned image sequences 1210 before enhancement. "Section 1," "Section 2," "Section 3," "Section 4," and "Section 5" in a fourth column 1204 of the table 1200 may refer to labels of an aligned image sequence 1220 after enhancement. The aligned image sequences 1210 before enhancement may be displayed in a second column 1202 of the table 1200, which correspond to the labels displayed in the first column 1201, respectively. For example, as shown in FIG. 12, five aligned image sequences 1210 before enhancement may be selected from the plurality of aligned image sequences and correspond to "Section 1," "Section 2," "Section 3," "Section 4," and "Section 5" in the first column 1201, respectively. The aligned image sequences 1220 after enhancement may be displayed in a third column 1203 of the table 1200, which correspond to the labels displayed in the fourth column 1204, respectively. For example, as shown in FIG. 12, five aligned image sequences 1220 after enhancement may be selected from the plurality of aligned image sequences and correspond to "Section 1," "Section 2," "Section 3," "Section 4," and "Section 5" in the fourth column 1204, respectively.

In some embodiments, a reconstructed image and/or a multi-layer image relating to the blood vessel (denoted by 1230) that supports switching image sequences may be displayed in a fifth column 1205 (e.g., a first row thereof) of the table 1200. The reconstructed image may include a 3D reconstructed image of a maximum density projection relating to the blood vessel. In some embodiments, intermediate calculation parameters 1240 that are used to calculate the enhancement level of the target tissue may be displayed in the fifth column 1504 (e.g., a second row thereof). For example, Table A illustrates exemplary intermediate calculation parameters. As shown in Table A, *R_{ef}* represents a mean value of the reference ROI before enhancement, and *R*_{*ef*1} represents a mean value of the reference ROI after enhancement. *W* represents a mean value of the region of the vascular wall before enhancement, and *W*₁ represents a mean value of the region of the vascular wall after enhancement. *P* represents a mean value of the region of the plaque before enhancement, and *P*₁ represents a mean value of the region of the plaque after enhancement. *R* represents a mean value of other target tissues before enhancement, and *R*₁ represents a mean value of other target tissues after enhancement. *R_{efER}* represents an enhancement degree of the reference ROI, which may be determined based on *R_{ef}* and *R*_{*ef*1}, such as by dividing *R*_{*ef*1} by *R_{ef}* (i.e., *R*_{*ef*1}/*R_{ef}*). *W_{ER}* represents an enhancement degree of the region of the vascular wall, which may be determined based on *W* and *W*₁, such as by dividing *W*₁ by *W* (i.e., *W*₁/*W). P_{ER}* represents an enhancement degree of the region of the plaque, which may be determined based on *P* and *P*₁, such as by dividing *P*₁ by *P* (i.e., *P*₁/*P*). *R_{ER}* represents an enhancement degree of other target tissues, which may be determined based on *R* and *R*₁, such as by dividing *R*₁ by *R*. An enhancement level of the vascular wall may be determined based on *W_{ER}* and *R_{efER},* such as by dividing *W_{ER}* by *R_{efER}.* An enhancement level of the plaque may be determined based on *P_{ER}* and *R_{efER},* such as by dividing *P_{ER}* by *R_{efER}.* An enhancement level of other target tissues may be determined based on *R_{ER}* and *R_{efER},* such as by dividing *R_{ER}* by *R_{efER}*. In some embodiments, *W* and *P* may be used to represent the first intensity in FIG. 10 achieved by operation 1010, and *W*₁ and *P*₁ may be used to represent the second intensity in FIG. 10 achieved by operation 1020.

**Table A**

| | mean value before enhancement | mean value after enhancement | enhancement degree | enhancement level |
|---|---|---|---|---|
| reference ROI | *R_{ef}* | *R*_{*ef*1} | *R_{efER}* (*R*_{*ef*1}/*R_{ef}*) | / |
| vascular wall | *W* | *W*₁ | *W_{ER}* (*W*₁/*W*) | *W_{ER}*/*R_{efER}* |
| plaque | *P* | *P*₁ | *P_{ER}* (*P*₁/*P*) | *P_{ER}*/*R_{efER}* |
| other target tissues | *R* | *R*₁ | *R_{ER}* (*R*₁/*R*) | *R_{ER}*/*R_{efER}* |

In some embodiments, the processing device 110 (e.g., the determining module 430) may determine a category that the enhancement level of the target tissue belongs to based on the enhancement degree (e.g., *W_{ER}, P_{ER},* etc.) of the region of the target tissue (e.g., the first enhancement degree of the region of the target tissue as described in FIG. 6) and the enhancement degree (e.g., *R_{efER}*) of the reference ROI (e.g., the second enhancement degree of the reference ROI as described in FIG. 6). In some embodiments, the category may include a significant enhancement level, a mild enhancement level, and no enhancement. The processing device 110 may determine the category of the enhancement level of the target tissue by comparing the first enhancement degree with the second enhancement degree. For example, the processing device 110 may determine the category of the enhancement level of the target tissue as the significant enhancement level in response to determining that the first enhancement degree is greater or equal to the second enhancement degree. As another example, the processing device 110 may determine the category of the enhancement level of the target tissue as the mild enhancement level in response to determining that the first enhancement degree is less than the second enhancement degree. As another example, the processing device 110 may determine the category of the enhancement level of the target tissue as no enhancement in response to determining that the first intensity (e.g., *W, P,* etc.) of the region of the target tissue before enhancement is equal to (or substantially equal to) the second intensity (e.g., *W*₁, *P*₁, etc.) of the region of the target tissue after enhancement.

The processing device 110 may determine the aligned image sequence(s) before enhancement and the aligned image sequence(s) after enhancement of the subject or a portion thereof based on the image registration, and determine the enhancement level of the target tissue (e.g., the vascular wall, the plaque, etc.) based on the aligned image sequence(s) before enhancement and the aligned image sequence(s) after enhancement, thereby avoiding a negative effect of a motion artifact on the enhancement level of the target tissue and improving an accuracy of the enhancement level of the target tissue. In addition, a manual interference may be avoided to improve an accuracy of determining the enhancement level of the plaque, and a time of determining the enhancement level of the plaque may also be shortened.

FIG. 13 is a schematic diagram illustrating an exemplary report of a target tissue according to some embodiments of the present disclosure.

In some embodiments, the processing device 110 (e.g., the determining module 430) may determine a report 1300 of the target tissue based on the plurality of aligned image sequences, the region of the target tissue, the reference ROI, and/or the enhancement level of the target tissue. The report 1300 of the target tissue may include an electronic report, a paper report, or the like, or any combination thereof. It should be noted that, the report 1300 in FIG. 13 is for illustrative purposes, which is not intended to limit the layout thereof. The report 1300 may be in any other layout.

In some embodiments, the processing device 110 may determine the report 1300 of the target tissue by displaying the plurality of aligned image sequences, the region of the target tissue, the reference ROI, and/or the enhancement level of the target tissue on a same interface. The plurality of aligned image sequences, the region of the target tissue, the reference ROI, and the enhancement level of the target tissue may be arranged in various layouts in the report 1300 without overlapping with each other. For instance, the report 1300 of the target tissue may be displayed in a layout as illustrated in FIG. 13. A first row of the report 1300 may include a plurality of reporting items (e.g., an object 1301, an image 1302, a parameter 1303, and a description 1304). The object 1301 may include the reference ROI (e.g., the pituitary stalk 1305), the region of the target tissue (e.g., the vascular wall 1306 and the plaque 1307), and other target tissues 1308. Taking the plaque as an example, when the object 1301 refers to the plaque 1307, the image 1302 may refer to a multi-layer image of a section of the plaque 1307, the parameter 1303 may include a plaque volume, pixel values before and after enhancement, enhancement degree, and enhancement level, and the description 1304 may include a plaque size and an enhancement situation.

In some embodiments, the report 1300 of the target tissue may also include the first intensity of the region of the target tissue before enhancement, the second intensity of the region of the target tissue after enhancement, the third intensity of the reference ROI before enhancement, the fourth intensity of the reference ROI after enhancement, or any other parameter relating to the target tissue and/or the reference ROI. For example, if the target tissue is a vascular wall, as the vascular wall is in the vascular lumen, the report 1300 may include parameter relating to the vascular wall and the vascular lumen.

FIG. 14 is a flowchart illustrating an exemplary process for one or more target parameters according to some embodiments of the present disclosure. In some embodiments, a process 1400 may be executed by the image analysis system 100. For example, the process 1400 may be implemented as a set of instructions (e.g., an application) stored in a storage device (e.g., the storage device 150, the storage 220, and/or the storage 390). In some embodiments, the processing device 110 (e.g., the processor 210 of the computing device 200, the CPU 340 of the mobile device 300, and/or one or more modules illustrated in FIG. 4) may execute the set of instructions and may accordingly be directed to perform the process 1400. The process 1400 may include following operations. In some embodiments, at least a portion of the operation 540 in FIG. 5 may be achieved by the process 1400.

In 1410, the processing device 110 (e.g., the determining module 430) may determine at least one region including a target tissue (e.g., a target blood vessel) by processing a first aligned image sequence and a second aligned image sequence.

In some embodiments, the first aligned image sequence and the second aligned image sequence may be aligned image sequences of the plurality of aligned image sequences as described in FIG. 5. More details about the plurality of aligned image sequences and the target blood vessel may be found elsewhere in the present disclosure (e.g., FIG. 5, FIG. 11, and related descriptions thereof).

The first aligned image sequence may refer to an image series representing blood flow information of the target blood vessel. For example, the first aligned image sequence may include an image sequence acquired using a 4D flow MRI sequence. The 4D flow MRI sequence may refer to a time-resolved 3D magnetic resonance phase contrast imaging sequence. An image (e.g., the first aligned image sequence) acquired using the 4D flow MRI sequence may represent multi-directional blood flow information of the target blood vessel. For example, the 4D flow MRI sequence may include phase sequences and amplitude sequences along three directions (e.g., three perpendicular dimensions in 3D space). The phase sequences along the three directions may be used to represent the blood flow information of the target blood vessel along the three directions. The amplitude sequences may be used to represent amplitudes of the blood flow information, and compensate for the blood flow information in a certain direction. The image acquired using the 4D flow MRI sequence may comprehensively reflect the blood flow information based on different pixels in the image. For example, the image acquired using the 4D flow MRI sequence may represent, by the different pixels of the image, the blood flow, the flow rate, the impulse of the blood flow to the vascular wall, or the like, or any combination thereof.

The second aligned image sequence may refer to an image series representing structure information (e.g., wall information) of the target blood vessel. For example, the second aligned image sequence may include an image sequence acquired using a T1 imaging sequence, a T1CE imaging sequence, a T2 imaging sequence, etc. The T1CE imaging sequence may refer to an imaging sequence that examines a blood vessel after infusion/injection with drugs. A bright region in an image that is acquired using the T1CE imaging sequence may represent that the region is rich in blood supply. More details about the T1 sequence may be found elsewhere in the present disclosure (e.g., FIG. 5 and related descriptions thereof).

In some embodiments, the processing device 110 may determine the at least one region including the target blood vessel by processing the first aligned image sequence and the second aligned image sequence. More details about determining the at least one region including the target blood vessel may be found elsewhere in the present disclosure (e.g., FIG. 5 and related descriptions thereof).

In 1420, the processing device 110 (e.g., the determining module 430) may determine blood flow information and wall information of the target blood vessel based on the at least one region including the target blood vessel.

The blood flow information may refer to related information about blood flow of the target blood vessel. For example, the blood flow information may include data information (e.g., blood flow, flow rate, etc.) about the blood flow of the 4D Flow MRI sequence. The wall information may refer to related information about an image sequence of the vascular wall in the plurality of aligned image sequences. For example, the wall information may include data information (e.g., a thickness of the vascular wall, etc.) about the vascular wall in the T1 sequence and/or the T1CE sequence

In some embodiments, the processing device 110 may determine the blood flow information of the target blood vessel based on the at least one region including the target blood vessel by processing the first aligned image sequence. For example, the processing device 110 may obtain the reference image sequence of the plurality of image sequences. The processing device 110 may determine/extract a centerline of the target blood vessel in the reference image sequence. The processing device 110 may also determine a centerline of the target blood vessel in the first aligned image sequence that is aligned with the reference image sequence. The processing device 110 may determine the blood flow information of the target blood vessel by segmenting, based on the centerline of the target blood vessel in the first aligned image sequence, the target blood vessel from the first aligned image sequence.

As used herein, a centerline may refer to a line representing a center position of a blood vessel, such as the centerline of the target blood vessel in the reference image sequence. In some embodiments, the processing device 110 may extract the centerline of the target vessel (e.g., the target blood vessel) in the reference image sequence based on one or more related technologies and/or algorithms (e.g., a shortest path technology, a topology refinement algorithm, a Dijkstra algorithm, etc.). The processing device 110 may determine, based on the centerline of the target blood vessel in the reference image sequence, the centerline of the target blood vessel in the first aligned image sequence that is aligned with the reference image sequence. For example, the processing device 110 may determine the position of the centerline of the target blood vessel in the reference image sequence as a reference position. The processing device 110 may determine the centerline of the target blood vessel in the first aligned image sequence based on the reference position. In some embodiments, the processing device 110 may determine, based on the centerline of the target blood vessel in the first aligned image sequence, the blood flow information (e.g., the blood flow, the flow rate, etc.) of the first aligned image sequence by using a vessel segmentation algorithm (e.g., an edge detection enhanced filtering algorithm based on Hessian matrix, a matching filtering algorithm, an adaptive contrast enhancement algorithm, etc.).

In some embodiments, the processing device 110 may determine the wall information of the target blood vessel based on the at least one region including the target blood vessel by processing the second aligned image sequence. For example, the processing device 110 may determine a centerline of the target blood vessel in the second aligned image sequence that is aligned with the reference image sequence. The processing device 110 may determine the wall information (e.g., a region of the vascular wall, a thickness of the vascular wall, etc.) about the second aligned image sequence based on the centerline of the target vessel in the second aligned image sequence. In some embodiments, the processing device 110 may determine lumen information about the second aligned image sequence based on the wall information. For example, the processing device 110 may determine the lumen in the second aligned image sequence based on an inner contour of the target blood vessel included in the wall information, and determine the lumen information based on the lumen in the second aligned image sequence.

In some embodiments, the processing device 110 may determine the wall information by extracting the vascular wall in the second aligned image sequence through, e.g., an automatic manner. The processing device 110 may determine the lumen information based on the inner contour of the target blood vessel included in the wall information. In some embodiments, the processing device 110 may adjust the wall information about the second aligned image sequence based on actual needs. For example, the processing device 110 may enlarge and/or shrink the vascular wall of the target blood vessel, etc.

In 1430, the processing device 110 (e.g., the determining module 430) may determine the one or more target parameters based on the blood flow information and the wall information.

In some embodiments, the processing device 110 may determine the one or more target parameters based on the blood flow information about the first aligned image sequence and the wall information about the second aligned image sequence. For example, the processing device 110 may determine the target blood vessel by performing an image registration (e.g., the non-rigid image registration, the rigid image registration, the affine registration, etc.) on the first aligned image sequence and the second aligned image sequence based on the blood flow information and the wall information. Further, the processing device 110 may determine a portion of the one or more target parameters (e.g., the flow quantity, the flow rate, the wall shear force, etc.) based on the blood flow information of the target blood vessel. The processing device 110 may determine another portion of the one or more target parameters (e.g., the wall thickness, etc.) based on the wall information of the target blood vessel. For example, the processing device 110 may determine the flow rate (e.g., an average flow rate, a peak flow rate, etc.) and the flow quantity (e.g., a bi-directional flow quantity, an average flow quantity, etc.) of a section of the target blood vessel based on the blood flow information about the first aligned image sequence. As another example, the processing device 110 may determine the wall thickness (e.g., a thickness of the vascular wall, a standard wall index, etc.) based on the wall information about the second aligned image sequence. More details about the one or more target parameters may be found elsewhere in the present disclosure (e.g., FIG. 5, FIG. 6, FIG. 10, FIG. 14, and related descriptions thereof).

It should be noted that the above description regarding the process 1400 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 1400 may be omitted, and/or one or more additional operations may be added.

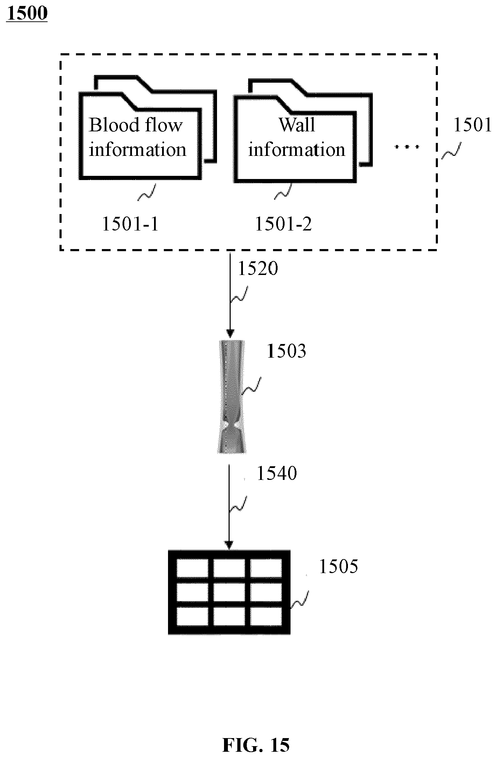

FIG. 15 is schematic diagram illustrating an exemplary process for a determination of one or more target parameters according to some embodiments of the present disclosure. In some embodiments, a process 1500 may be executed by the image analysis system 100. For example, the process 1500 may be implemented as a set of instructions (e.g., an application) stored in a storage device (e.g., the storage device 150, the storage 220, and/or the storage 390). In some embodiments, the processing device 110 (e.g., the processor 210 of the computing device 200, the CPU 340 of the mobile device 300, and/or one or more modules illustrated in FIG. 4) may execute the set of instructions and may accordingly be directed to perform the process 1500. The process 1500 may include following operations. In some embodiments, operation 1430 in FIG. 14 may be achieved by the process 1500.

In 1520, the processing device 110 (e.g., the determining module 430) may determine a reconstructed image of the target blood vessel based on the blood flow information and the wall information.

As used herein, the reconstructed image may refer to an image obtained by processing multiple image sequences based on an image reconstruction algorithm. More details about the image reconstruction algorithm may be found elsewhere in the present disclosure (e.g., FIG. 5 and related descriptions thereof). The reconstructed image may include a 3D visualized reconstructed image, a carved projection reformation (CPR) image, a multi-planar reconstructed (MPR) image, etc.

In some embodiments, the 3D visualized reconstructed image may refer to a reconstructed image displayed in a 3D perspective image. A user may determine a state of the target blood vessel clearly based on the 3D visualized reconstructed image. The CPR image may refer to an image obtained based on a curved projection reformation technology/algorithm. The CPR technology may stretch and straightening a twisted, shortened, and/or overlapped blood vessel, so that the blood vessel may be displayed in a same plane. The CPR technology may be an optimization of the MPR technology. In some embodiments, the CPR image may be an image representing the target blood vessel from multiple views through various manners. For example, the CPR image may be an MPR image rotating with 360 degrees.

In some embodiments, the reconstructed image may represent the target blood vessel through various manners. For example, as shown in FIG. 17, a reconstructed image 1503 of the target blood vessel includes a vascular wall 1503-1 of the target blood vessel, a lumen 1503-2 of the target blood vessel, an ROI 1503-3, a first plane 1602 (e.g., a stenosis plane), a reference plane 1604, etc. An inside of the lumen 1503-2 may represent parameters such as different flow rates through different rendering manners (e.g., different pixels, different colors, different streamlines, etc.). The reconstructed image 1503 of the target blood vessel may represent the one or more target parameters of the target blood vessel and a relationship among the vascular wall 1503-1, the lumen 1503-2, the ROI 1503-3, the first plane 1602 (e.g., a stenosis plane), the reference plane 1605, etc. It should be noted that FIG. 17 is an exemplary manner to represent the reconstructed image of the target blood vessel. In some embodiments, the reconstructed image of the target blood vessel may be represented in other feasible manners, which is not limited herein.

In some embodiments, the processing device 110 may determine the reconstructed image of the target blood vessel based on the blood flow information and the wall information by using a multi planar reconstruction (MPR) algorithm, a curved projection reformation (CPR) algorithm, etc. As shown in FIG. 15, information 1501 may include blood flow information 1501-1 and wall information 1501-2. The blood flow information 1501-1 may include multiple blood flow information about the first aligned image sequence (e.g., acquired using the 4D flow MRI sequence). For example, the blood flow information 1501-1 may include blood flow information about multiple images in the first aligned image sequence corresponding to three phase sequences and/or amplitude sequences. The wall information 1501-2 may include multiple wall information about the second aligned image sequence. For example, the wall information 1501-2 may include wall information about multiple images in the second aligned image sequence (e.g., the T1 sequence, the T1CE sequence, etc.). The processing device 110 may determine the reconstructed image of the target blood vessel by fusing and reconstructing the information 1501 based on an image reconstruction algorithm such as the CPR algorithm. In some embodiments, the processing device 110 may determine an MPR image relating to the target blood vessel based on the MPR technology. In some embodiments, the processing device 110 may determine a CPR image relating to the target blood vessel by recombining the blood flow information 1501-1, the lumen information (not shown in FIG. 15), and/or the wall information 1501-2 along the centerline of the target blood vessel in the first aligned image sequence. In some embodiments, a sectional view of the target blood vessel may be obtained based on the CPR image. The processing device 110 may obtain related information (e.g., the morphology of the target blood vessel, the vascular wall, etc.) about the target blood vessel based on the sectional view.

In 1540, the processing device 110 (e.g., the determining module 430) may determine the one or more target parameters based on the reconstructed image.

In some embodiments, the processing device 110 may determine one or more target parameters 1505 of the target blood vessel based on the related information (e.g., hemodynamic information about the blood flow, morphological information about the blood vessel, etc.) about the reconstructed image.

In some embodiments, the processing device 110 may store the one or more target parameters and the reconstructed image of the target blood vessel. The processing device 110 may further transmit related information, such as the one or more target parameters and the reconstructed image of the target blood vessel, to a user in a form of report (e.g., the report 1300).

According to some embodiments of the present disclosure, the reconstructed image may display the target blood vessel more clearly and directly. A qualitative and quantitative analysis of the target blood vessel may be achieved by estimating the wall thickness, the ROI (e.g., a region of plaque, a region of aneurysm, etc.), quantitative parameters of a stenosis plane, hemodynamic parameters the blood flow (e.g., the flow quantity, the flow rate, the wall shear force, etc.), or the like, or any combination thereof, thereby achieving a comprehensive analysis of a state of a stenosis plane, a region of plaque, a region of aneurysm, etc., of the target blood vessel.

According to some embodiments, the processing device 110 may determine the one or more target parameters of the target blood vessel based on the blood flow information and the wall information by processing the plurality of aligned image sequences, thereby achieving a qualitative and quantitative analysis of the target blood vessel based on kinetic parameters, morphological parameters, etc., and improving an accuracy of image analysis.

It should be noted that the above description regarding the process 1500 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 1500 may be omitted, and/or one or more additional operations may be added.

In some embodiments, the processing device 110 (e.g., the determining module 430) may extract one or more masks (e.g., a blood flow mask, a lumen mask, and a wall mask) from the blood flow information and the wall information for determining the one or more target parameters. As used herein, a mask operation may refer to a bitwise operation on a target field based on a string of binary code, which may mask a current input bit. In some embodiments, an extraction of the blood flow information and the wall information may refer to mask a region of the blood flow, the lumen, the vascular wall, etc. The blood flow mask may refer to mask the region of the blood flow. The lumen mask may refer to mask the region of the lumen. The wall mask may refer to mask the region of the vascular wall.

In some embodiments, the region of the blood flow, the region of the lumen, and the region of the vascular wall may be represented as different values in corresponding masks. For example, the region of the blood flow may be represented as "-1" in the blood flow mask, the region of the lumen may be represented as "0" in the lumen mask, and the region of the vascular wall may be represented as "1" in the vascular wall mask.

In some embodiments, the processing device 110 may determine the blood flow information about the first aligned image sequence (e.g., acquired using the 4D flow MRI sequence, etc.) and mask the blood flow information. For example, the processing device 110 may replace the region relating to the blood flow information with a specific value (e.g., -1). In some embodiments, the processing device 110 may determine the lumen information and the wall information about the second aligned image sequence (e.g., acquired using the T1 imaging sequence, the T1CE imaging sequence, etc.) and mask the lumen information and the wall information. For example, the processing device 110 may replace the region relating to the lumen information with a specific value (e.g., 0). As another example, the processing device 110 may replace the region relating to the wall information with a specific value (e.g., 1).

In some embodiments, the processing device 110 may mask the blood flow information, the lumen information, and the wall information in various manners. For example, the processing device 110 may mask the region of blood flow, the region of lumen, or the region of vascular wall based on a same number, vector, symbol, text, etc. The processing device 110 may set a masking manner based on actual needs, which is not limited herein.

In some embodiments, the processing device 110 may determine the target blood vessel by using the image registration (e.g., the non-rigid registration, the rigid registration, the affine registration, etc.) based on the blood flow mask of the first aligned image sequence, and the lumen mask and the wall mask of the second aligned image sequence. The processing device 110 may determine a portion of the one or more target parameters (e.g., the flow quantity, the flow rate, the wall shear force, etc.) of the target blood vessel based on the blood flow information. The processing device 110 may also determine another portion of the one or more target parameters (e.g., the wall thickness, etc.) of the target blood vessel based on the wall information.

According to some embodiments, the processing device 110 may determine the one or more target parameters of the target blood vessel by masking the blood flow information, the lumen information, and the wall information, thereby improving a registration performance of different images and an accuracy of the registration of the target blood vessel, so that an accuracy of the one or more target parameters of the target blood vessel may also be improved to facilitate a qualitative and quantitative analysis of the target blood vessel.

In some embodiments, the processing device 110 may export/display the one or more target parameters of the target blood vessel in a form of table. For example, a first column of the table may include the one or more target parameters (e.g., the flow quantity, the flow rate, the wall shear force, the wall thickness, etc.), and a first row of the table may include a plurality of regions (e.g., a maximum sectional area of the region of plaque, the first plane (e.g., the stenosis plane), the reference plane, a maximum sectional area of the region of aneurysm, etc.) of the target blood vessel, so that the one or more target parameters of the target blood vessel may correspond to the plurality of regions, respectively.

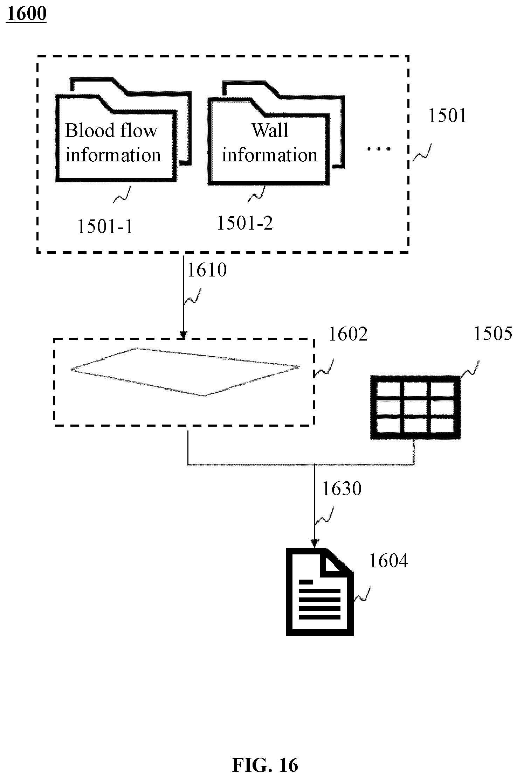

FIG. 16 is schematic diagram illustrating an exemplary process for a determination of a parameter of a first plane according to some embodiments of the present disclosure. In some embodiments, a process 1600 may be executed by the image analysis system 100. For example, the process 1600 may be implemented as a set of instructions (e.g., an application) stored in a storage device (e.g., the storage device 150, the storage 220, and/or the storage 390). In some embodiments, the processing device 110 (e.g., the processor 210 of the computing device 200, the CPU 340 of the mobile device 300, and/or one or more modules illustrated in FIG. 4) may execute the set of instructions and may accordingly be directed to perform the process 1600. The process 1600 may include following operations.

In 1610, the processing device 110 (e.g., the determining module 430) may determine a first plane of the target blood vessel based on the blood flow information and the wall information.

In some embodiments, the first plane may include a stenosis plane of the target blood vessel. A lumen of the first plane may be less than a lumen of another region in the target blood vessel. The target blood vessel may include a plurality of first planes. In some embodiments, the target blood vessel may include the first plane, a reference plane, etc. The reference plane may refer to a plane of a normal region of the target blood vessel. For example, as shown in FIG. 17, the reconstructed image 1503 of the target blood vessel may include a plurality of first planes 1602 (e.g., a first plane 1602-1, a first plane 1602-2, etc.,) and a reference plane 1605 which is a plane of a normal region.

In some embodiments, the processing device 110 may determine the first plane of the target blood vessel based on multiple information about the first aligned image sequence and the second aligned image sequence. The multiple information may include the blood flow information (e.g., the blood flow information 1501-1, etc.) about the first aligned image sequence and the wall information (e.g., the wall information 1501-2, etc.) about the second aligned image sequence. In some embodiments, the processing device 110 may determine the first plane (e.g., the stenosis plane) based on the blood flow information about the first aligned image sequence (e.g., acquired using the 4D flow MRI sequence) and the sectional information about the second aligned image sequence (e.g., the T1 sequence) in an automatic manner. For example, the processing device 110 may determine a target plane or a plurality of planes of a target region as the first plane when the flow quantity and/or the flow rate of the target plane or the target region are determined to be less than a first threshold based on the blood flow information (e.g., the flow quantity, the flow rate, etc.) about the first aligned image sequence (acquired using the 4D flow MRI sequence) and a diameter of a lumen of the target plane or the target region is determined to be less than a second threshold based on sectional information of the second image sequence (acquired using the T1 imaging sequence). In some embodiments, the first threshold may refer to a flow quantity threshold and/or a flow rate threshold. The flow quantity and/or the flow rate below the flow quantity threshold and/or the flow rate threshold may indicate that a flowing of the blood may be blocked, so that the flow quantity and/or the flow rate become smaller. In some embodiments, the second threshold may be a diameter threshold of a lumen. The lumen diameter below the second threshold may indicate that the lumen becomes narrow.

In some embodiments, the processing device 110 may transmit the first plane (e.g., the stenosis plane) 1602 determined by a user or system to the plurality of aligned image sequences (e.g., acquired using the TOF imaging sequence) in an automatic manner based on the registration relationship between the plurality of aligned image sequences. In some embodiments, the processing device 110 may determine the first plane 1602 based on a position of a first plane relating to the first aligned image sequence and a position of a first plane relating to the second aligned image sequence. In some embodiments, the processing device 110 may determine a position of the first plane 1602 based on a visualized technology (e.g., a flow rate diagram, a vector diagram, a streamline, a trace diagram, etc.) of the 4D flow MRI. In some embodiments, the processing device 110 may adjust the first plane 1602 based on actual needs. The processing device 110 may increase a count of the first planes, change a position of the first plane, or the like, or any combination thereof. For example, the processing device 110 may adjust the first plane upward, downward, etc.

In some embodiments, the processing device 110 may determine a confidence level of the determined first plane (e.g., the first plane 1602). The confidence level of the first plane may refer to a reliable degree of the first plane determined by the processing device 110. In some embodiments, the confidence level of the first plane may include a first confidence level and a second confidence level. The first confidence level may refer to a credibility of the blood flow information and the wall information. In some embodiments, the first confidence level may be a constant confidence level. The processing device 110 may determine the first confidence level based on a credibility of a device (e.g., the processor 210 of the computing device 200, the CPU 340 of the mobile device 300, and/or one or more modules illustrated in FIG. 4) that is used to obtain the first confidence level. The second confidence level may refer to a credibility of the reconstructed image of the target blood vessel. In some embodiments, the processing device 110 may determine the second confidence level based on historical data.

In some embodiments, the processing device 110 may determine the confidence level of the first plane by multiplying the first confidence level by the second confidence level. In some embodiments, the processing device 110 may adjust the first plane based on the confidence level of the first plane. For example, the processing device 110 may determine whether a position of the first plane is adjusted based on the confidence level of the first plane and a preset level or a preset range. The preset level and/or the preset range may be used to represent that a position of the first plane is normal or suitable. The preset level and/or the preset range may be determined by a manual setting or a system setting. In response to determining that the confidence level of the first plane is less than a preset level or does not locate in a preset range, the processing device 110 may increase the count of the first planes and/or change the position of the first plane, such as adjusting the first plane upward or downward or the like.

In 1630, the processing device 110 (e.g., the determining module 430) may determine one or more parameters of the first plane based on the first plane and the one or more target parameters.

The one or more parameters of the first plane may refer to one or more parameters representing information of the first plane. For example, the one or more parameters of the first plane may include a flow quantity relating to the first plane, a flow rate relating to the first plane, a wall thickness of the vascular wall relating to the first plane, a wall shear force relating to the first plane, etc.

In some embodiments, the processing device 110 may determine one or more parameters 1604 based on the first plane 1602 and one or more target parameters 1505 of the target blood vessel. For example, the processing device 110 may determine the flow rate (e.g., an average flow rate, a peak flow rate, etc.) relating to the first plane, the flow quantity relating to the first plane, the wall shear force relating to the first plane, etc., based on the position of the first plane and the one or more target parameters of the target blood vessel. In some embodiments, the processing device 110 may determine a relative stenosis ratio of the first plane of the target blood vessel based on one or more parameters of the reference plane and the one or more parameters of the first plane.

It should be noted that the above description regarding the process 1600 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 1600 may be omitted, and/or one or more additional operations may be added.

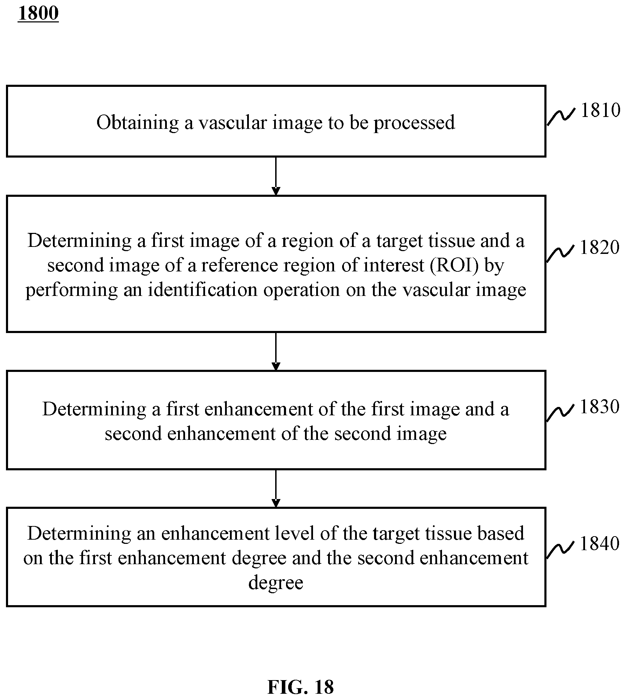

FIG. 18 is a flowchart illustrating an exemplary process for image analysis according to some embodiments of the present disclosure. In some embodiments, a process 1800 may be executed by the image analysis system 100. For example, the process 1800 may be implemented as a set of instructions (e.g., an application) stored in a storage device (e.g., the storage device 150, the storage 220, and/or the storage 390). In some embodiments, the processing device 110 (e.g., the processor 210 of the computing device 200, the CPU 340 of the mobile device 300, and/or one or more modules illustrated in FIG. 4) may execute the set of instructions and may accordingly be directed to perform the process 1800. The process 1800 may include following operations.

In 1810, the processing device 110 (e.g., the obtaining module 410) may obtain a vascular image to be processed. The vascular image to be processed may include an image sequence relating to a blood vessel or an aligned image sequence relating to the blood vessel. More descriptions of obtaining the vascular image to be processed may be found elsewhere in the present disclosure (e.g., operation 510 or the descriptions thereof).

In 1820, the processing device 110 (e.g., the determining module 430) may determine a first image of a region of a target tissue and a second image of a reference region of interest (ROI) by performing an identification operation on the vascular image. As used herein, the first image of the region of a target tissue may be similar to the region of the target tissue as described in operation 530. The second image of a region of the reference ROI may be similar to the region of the reference ROI as described in operation 530. More descriptions of determining the first image of the region of the target tissue and the second image of the reference region of interest (ROI) by performing the identification operation on the vascular image may be found elsewhere in the present disclosure (e.g., FIG. 5, FIG. 7, FIG. 8, FIG. 14, or the descriptions thereof).

In 1830, the processing device 110 (e.g., the determining module 430) may determine a first enhancement degree of the first image and a second enhancement degree of the second image. As used herein, the first enhancement degree may be similar to the first enhancement degree as described in operation 610. The second enhancement degree may be similar to the second enhancement degree as described in operation 620. More descriptions of determining the first enhancement degree of the first image and the second enhancement degree of the second image may be found elsewhere in the present disclosure (e.g., FIG. 6 or the descriptions thereof).

In 1840, the processing device 110 (e.g., the determining module 430) may determine an enhancement level of the target tissue based on the first enhancement degree and the second enhancement degree. As used herein, the enhancement level may be similar to the enhancement level as described in operation 630. More descriptions of determining an enhancement level of the target tissue based on the first enhancement degree and the second enhancement degree may be found elsewhere in the present disclosure (e.g., FIG. 6 or the descriptions thereof).

It should be noted that the above description regarding the process 1800 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 1800 may be omitted, and/or one or more additional operations may be added.

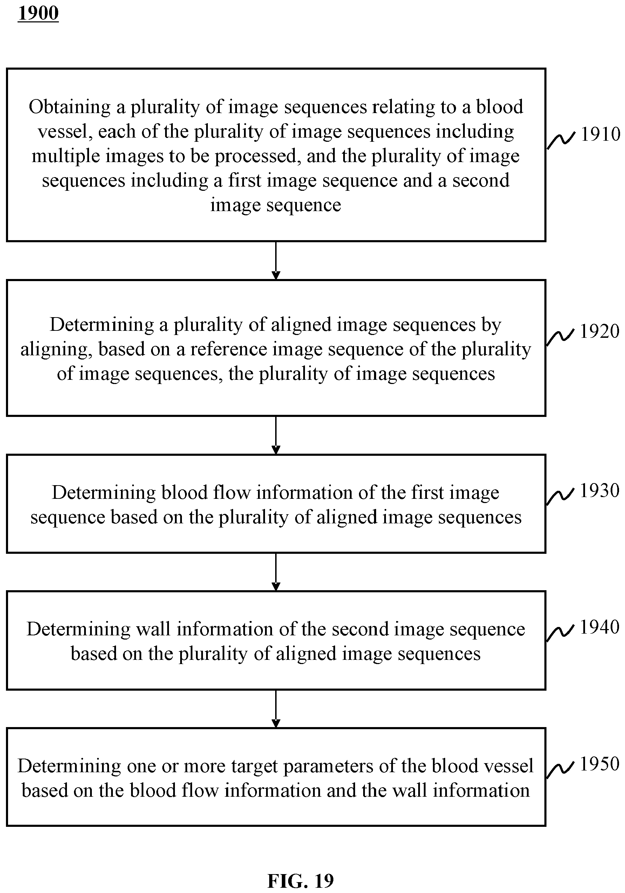

FIG. 19 is a flowchart illustrating an exemplary process for image analysis according to some embodiments of the present disclosure. In some embodiments, a process 1900 may be executed by the image analysis system 100. For example, the process 1900 may be implemented as a set of instructions (e.g., an application) stored in a storage device (e.g., the storage device 150, the storage 220, and/or the storage 390). In some embodiments, the processing device 110 (e.g., the processor 210 of the computing device 200, the CPU 340 of the mobile device 300, and/or one or more modules illustrated in FIG. 4) may execute the set of instructions and may accordingly be directed to perform the process 1900. The process 1900 may include following operations.

In 1910, the processing device 110 (e.g., the obtaining module 410) may obtain a plurality of image sequences relating to a blood vessel. Each of the plurality of image sequences may include multiple images to be processed, and the plurality of image sequences may include a first image sequence and a second image sequence. As used herein, the plurality of image sequences relating to the blood vessel may be similar to the plurality of image sequences relating to the blood vessel as described in operation 510. More descriptions of obtaining the plurality of image sequences relating to the target blood vessel may be found elsewhere in the present disclosure (e.g., FIG. 5 and FIG. 14 or the descriptions thereof).

In 1920, the processing device 110 (e.g., the determining module 430) may determine a plurality of aligned image sequences by aligning the plurality of image sequences based on a reference image sequence of the plurality of image sequences. As used herein, the plurality of aligned image sequences and the reference image sequence of the plurality of image sequences may be similar to the plurality of aligned image sequences and the reference image sequence of the plurality of image sequences as described in operation 520. More descriptions of determining the plurality of aligned image sequences by aligning the plurality of image sequences based on the reference image sequence of the plurality of image sequences may be found elsewhere in the present disclosure (e.g., FIG. 5 and FIG. 11 or the descriptions thereof).

In 1930, the processing device 110 (e.g., the determining module 430) may determine blood flow information of the first image sequence based on the plurality of aligned image sequences. As used herein, the blood flow information of the first image sequence may be similar to the blood flow information of the first aligned image sequence as described in operation 1420. More descriptions of determining the blood flow information of the first image sequence based on the plurality of aligned image sequences may be found elsewhere in the present disclosure (e.g., FIG. 14 or the descriptions thereof).

In 1940, the processing device 110 (e.g., the determining module 430) may determine wall information of the second image sequence based on the plurality of aligned image sequences. As used herein, the wall information of the second image sequence may be similar to the wall information of the second aligned image sequence as described in operation 1420. More descriptions of determining the wall information of the second image sequence based on the plurality of aligned image sequences may be found elsewhere in the present disclosure (e.g., FIG. 14 or the descriptions thereof).

In 1950, the processing device 110 (e.g., the determining module 430) may determine one or more target parameters of the blood vessel based on the blood flow information and the wall information. As used herein, the one or more target parameters of the blood vessel may be similar to the one or more target parameters as described in operation 530. More descriptions of determining the one or more target parameters of the target blood vessel based on the blood flow information and the wall information may be found elsewhere in the present disclosure (e.g., FIG. 5 and FIG. 14 or the descriptions thereof).

It should be noted that the above description regarding the process 1900 is merely provided for the purposes of illustration, and not intended to limit the scope of the present disclosure. For persons having ordinary skills in the art, multiple variations and modifications may be made under the teachings of the present disclosure. However, those variations and modifications do not depart from the scope of the present disclosure. In some embodiments, one or more operations of the process 1900 may be omitted, and/or one or more additional operations may be added.

## Claims

1. A method for image analysis, comprising:
obtaining a plurality of image sequences relating to a blood vessel, at least one of the plurality of image sequences being acquired using a black blood imaging sequence;
determining a plurality of aligned image sequences by aligning, based on a reference image sequence of the plurality of image sequences, the plurality of image sequences;
determining a target region based on the plurality of aligned image sequences; and
determining one or more target parameters based on the target region.

2. The method of claim 1, wherein the target region includes a region of a target tissue and a reference region of interest (ROI), the one or more target parameters include an enhancement level of the target tissue, and the determining the one or more target parameters based on the target region includes:
determining, based on the region of the target tissue, a first enhancement degree of the region of the target tissue;
determining, based on the reference ROI, a second enhancement degree of the reference ROI; and
determining the enhancement level of the target tissue based on the first enhancement degree and the second enhancement degree.

3. The method of claim 2, wherein determining, based on the region of the target tissue, the first enhancement degree of the region of the target tissue includes:
determining a first intensity of the region of the target tissue before enhancement;
determining a second intensity of the region of the target tissue after enhancement; and
determining, based on the first intensity and the second intensity, the first enhancement degree of the region of the target tissue.

4. The method of claim 2 or claim 3, wherein determining, based on the refence ROI, the second enhancement degree of the reference ROI includes:
determining a third intensity of the reference ROI before enhancement;
determining a fourth intensity of the reference ROI after enhancement; and
determining, based on the third intensity and the fourth intensity, the second enhancement degree of the reference ROI.

5. The method of any one of claims 2-4, wherein the target tissue includes a vascular wall and a plaque, and the target tissue is determined by:
determining the vascular wall by identifying the vascular wall from the plurality of aligned image sequences; and
determining the plaque by identifying the plaque from the vascular wall.

6. The method of any one of claims 2-5, wherein the reference ROI is determined by:
inputting the plurality of aligned image sequences into a segmentation model.

7. The method of any one of claims 2-6, wherein determining the enhancement level of the target tissue based on the first enhancement degree and the second enhancement degree includes:
determining the enhancement level by dividing the first enhancement degree by the second enhancement degree.

8. The method of any one of claims 1-7, wherein the target region includes a region of a target blood vessel, and the one or more target parameters include at least one of a flow quantity of the target blood vessel, a flow rate of the blood vessel, a wall thickness of the target blood vessel, or a wall shear force of the target blood vessel.

9. The method of claim 8, wherein the plurality of aligned image sequences includes a first aligned image sequence and a second aligned image sequence, and determining the one or more target parameters based on the target region includes:
determining at least one region including the target blood vessel by processing the first aligned image sequence and the second aligned image sequence;
determining blood flow information and wall information of the target blood vessel based on the at least one region including the target blood vessel; and
determining the one or more target parameters based on the blood flow information and the wall information.

10. The method of claim 9, wherein determining the one or more target parameters based on the blood flow information and the wall information includes:
determining a reconstructed image of the target blood vessel based on the blood flow information and the wall information; and
determining the one or more target parameters based on the reconstructed image.

11. The method of claim 9 or 10, further including:
determining a first plane of the target blood vessel based on the blood flow information and the wall information; and
determining one or more parameters of the first plane based on the first plane and the one or more target parameters.

12. The method of any one of claims 9-11, wherein determining the one or more target parameters based on the blood flow information and the wall information includes:
extracting a blood flow mask, a lumen mask, and a wall mask from the blood flow information and the wall information; and
determining the one or more target parameters based on the blood flow mask, the lumen mask, and the wall mask.

13. A system for image analysis, comprising:
an obtaining module configured to obtain a plurality of image sequences relating to a blood vessel, at least one of the plurality of image sequences being acquired using a black blood imaging sequence;
an aligning module configured to determine a plurality of aligned image sequences by aligning, based on a reference image sequence of the plurality of image sequences, the plurality of image sequences;
a determining module configured to
determine a target region based on the plurality of aligned image sequences; and
the determining module, configured to determine one or more target parameters based on the target region.

14. The system of claim 13, wherein the target region includes a region of a target tissue and a reference region of interest (ROI), the one or more target parameters include an enhancement level of the target tissue, and to determine the one or more target parameters based on the target region, the determining module is configured to:
determine, based on the region of the target tissue, a first enhancement degree of the region of the target tissue;
determine, based on the reference ROI, a second enhancement degree of the reference ROI; and
determine the enhancement level of the target tissue based on the first enhancement degree and the second enhancement degree.

15. A non-transitory computer readable medium including executable instructions, the instructions, when executed by at least one processor, causing the at least one processor to effectuate a method for image analysis, the method comprising:
obtaining a plurality of image sequences relating to a blood vessel, at least one of the plurality of image sequences being acquired using a black blood imaging sequence;
determining a plurality of aligned image sequences by aligning, based on a reference image sequence of the plurality of image sequences, the plurality of image sequences;
determining a target region based on the plurality of aligned image sequences; and
determining one or more target parameters based on the target region.
